# EUROPÄISCHE PATENTSCHRIFT

(11) **EP 1 263 745 B1**
(45) Veröffentlichungstag und Bekanntmachung des Hinweises auf die Patenterteilung: **19.05.2004**
(21) Anmeldenummer: 01905805.6
(22) Anmeldetag: 20.02.2001
(51) Int. Cl.: C07D 271/113, C07D 413/10, C07D 413/14, A61K 31/4245, A61P 3/10

(54) **SUBSTITUIERTE 3-PHENYL-5-ALKOXI-1,3,4-OXDIAZOL-2-ONE UND IHRE VERWENDUNG ZUR HEMMUNG DER HORMONSENSITIVEN LIPASE**
SUBSTITUTED 3-PHENYL-5-ALKOXI-1,3,4-OXDIAZOL-2-ONE AND USE THEREOF FOR INHIBITING HORMONE-SENSITIVE LIPASE
3-PHENYL-5-ALCOXI-1,3,4-OXDIAZOL-2-ONES SUBSTITUEES ET LEUR UTILISATION POUR INHIBER LA LIPASE SENSIBLE AUX HORMONES

(30) Priorität: 07.03.2000 DE 10010968; 18.01.2001 DE 10102265
(43) Veröffentlichungstag der Anmeldung: 11.12.2002
(73) Patentinhaber: Aventis Pharma Deutschland GmbH, 65929 Frankfurt am Main (DE)
(72) Erfinder: SCHOENAFINGER, Karl, 63755 Alzenau (DE); PETRY, Stefan, 65929 Frankfurt (DE); MUELLER, Guenter, 65843 Sulzbach (DE); BARINGHAUS, Karl-Heinz, 61200 Wölfersheim (DE)
(86) Internationale Anmeldenummer: PCT/EP2001/001898
(87) Internationale Veröffentlichungsnummer: WO 2001/066531

(56) Entgegenhaltungen:
- WO-A-96/13264

## Beschreibung

Die Erfindung betrifft substituierte 3-Phenyl-5-alkoxi-1,3,4-oxdiazol-2-one, die eine hemmende Wirkung an der hormonsensitiven Lipase, HSL, zeigen.

Bestimmte 5-Alkoxi-1,3,4-oxdiazol-2-one mit einem ortho-substituierten Phenylring als Substituenten oder mit ankondensierten fünf- oder sechsgliedrigen Ringen besitzen anthelmintische (DE-A 26 04 110) und insektizide Wirkung (DE-A 26 03 877, EP-B 0 048 040, EP-B 0 067 471).

Bestimmte 5-Phenoxi-1,3,4-oxdiazol-2-one mit einem ortho-substituierten Phenylring als Substituenten zeigen endoparasitizide Wirkung (EP-A 0 419 918).

Bestimmte Azolderivate, insbesondere 1,3,4-Oxdiazol-2-one, besitzen eine antihyperglykämische Wirkung (WO-A 96 13264).

Ziel der Erfindung war es nun, Verbindungen zu finden, die eine hemmende Wirkung an der hormonsensitiven Lipase, HSL, zeigen.

Dies wurde erreicht mit den substituierten 3-Phenyl-5-alkoxi-1,3,4-oxdiazol-2-onen der allgemeinen Formel 1, 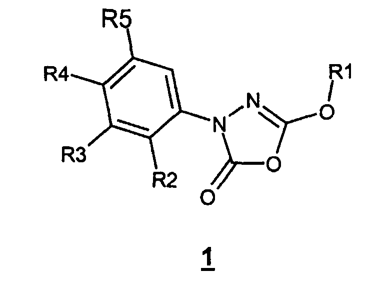 worin bedeuten:
- R¹: C₁-C₆-Alkyl, C₃-C₉-Cycloalkyl, wobei beide Gruppen durch Phenyl, C₁-C₄-Alkyloxy, S-C₁-C₄-Alkyl, N(C₁-C₄-Alkyl)₂ ein- oder mehrfach substituiert sein können und Phenyl wiederum durch Halogen, C₁-C₄-Alkyl, C₁-C₄-Alkyloxy, Nitro, CF₃, ein- oder mehrfach substituiert sein kann; und
- R², R³, R⁴ und R⁵: unabhängig voneinander Wasserstoff, Halogen, Nitro, C₁-C₄-Alkyl, C₁-C₉-Alkyloxy;
C₆-C₁₀-Aryl-C₁-C₄-alkyloxy, C₆-C₁₀-Aryloxy, C₆-C₁₀-Aryl, C₃-C₈-Cycloalkyl oder O-C₃-C₈-Cycloalkyl, die durch Halogen, CF₃, C₁-C₄-Alkyloxy oder C₁-C₄-Alkyl einfach, doppelt oder dreifach substituiert sein können;
2- Oxo-pyrrolidin-1-yl, 2,5-Dimethylpyrrol-1-yl oder NR⁶-A-R⁷,
mit der Maßgabe, dass R², R³, R⁴ und R⁵ nicht gleichzeitig Wasserstoff bedeuten und mindestens einer der Reste R², R³, R⁴ oder R⁵ für den Rest 2-Oxo-pyrrolidin-1-yl, 2,5-Dimethylpyrrol-1-yl oder NR⁶-A-R⁷ steht, mit
- R⁶: Wasserstoff, C₁-C₄-Alkyl oder C₆-C₁₀-Aryl-C₁-C₄-alkyl, wobei Aryl durch Halogen, CF₃, C₁-C₈-Alkyloxy oder C₁-C₄-Alkyl substituiert sein kann;
- A: eine einfache Bindung, COₙ, SOₙ oder CONH;
- n: 1 oder 2;
- R⁷: Wasserstoff;
C₁-C₁₈-Alkyl oder C₂-C₁₈-Alkenyl, die ein- bis dreifach durch C₁-C₄-Alkyl, Halogen, CF₃, C₁-C₄-Alkyloxy, N(C₁-C₄-Alkyl)₂, -COOH, C₁-C₄-Alkyloxycarbonyl, C₆-C₁₂-Aryl, C₆-C₁₂-Aryloxy, C₆-C₁₂-Arylcarbonyl, C₆-C₁₀-Aryl-C₁-C₄-alkyloxy oder Oxo substituiert sein können, wobei Aryl wiederum durch Halogen, C₁-C₄-Alkyl, Aminosulfonyl oder Methylmercapto substituiert sein kann;
C₆-C₁₀-Aryl-C₁-C₄-alkyl, C₅-C₈-Cycloalkyl-C₁-C₄-alkyl, C₅-C₈-Cycloalkyl, C₆-C₁₀-Aryl-C₂-C₆-alkenyl, C₆-C₁₀-Aryl, Diphenyl, Diphenyl-C₁-C₄-alkyl, Indanyl, die durch C₁-C₁₈-Alkyl, C₁-C₁₈-Alkyloxy, C₃-C₈-Cycloalkyl, COOH, Hydroxy, C₁-C₄-Alkylcarbonyl, C₆-C₁₀-Aryl-C₁-C₄-alkyl, C₆-C₁₀-Aryl-C₁-C₄-alkyloxy, C₆-C₁₀-Aryloxy, Nitro, Cyano, C₆-C₁₀-Aryl, Fluorsulfonyl, C₁-C₆-Alkyloxycarbonyl, C₆-C₁₀-Arylsulfonyloxy, Pyridyl, NHSO₂-C₆-C₁₀-Aryl, Halogen, CF₃ oder OCF₃ ein- oder zweifach substituiert sein können, wobei Alkyl nochmals durch C₁-C₄-Alkyloxycarbonyl, CF₃ oder Carboxy substituiert sein kann und Aryl durch Halogen, CF₃ oder C₁-C₄-Alkyloxy; oder die Gruppe Het-(CH₂)ᵣ-,
mit r = 0, 1, 2 oder 3 und Het = gesättigter oder ungesättigter 5-7-gliedriger Heterocyclus, der benzoanelliert und mit C₁-C₄-Alkyl, C₆-C₁₀-Aryl, Halogen, C₁-C₄-Alkyloxy, C₁-C₄-Alkyloxycarbonyl, C₆-C₁₀-Aryl-C₁-C₄-alkyl, C₆-C₁₀-Aryl-C₁-C₄-alkylmercapto oder Nitro substituiert sein kann, wobei benzoanelliertes Aryl wiederum durch Halogen, C₁-C₄-Alkyloxy oder CF₃ und Alkyl in Arylalkyl durch Methoxy und CF₃ substituiert sein kann,
sowie deren pharmakologisch verträglichen Salzen und Säureadditionssalzen.

Halogen steht für Fluor, Chlor, Brom, bevorzugt für Fluor und Chlor. Alkyl, Alkenyl, Alkyloxy usw. können verzweigt oder unverzweigt sein.

Bevorzugt sind Verbindungen der Formel 1 worin bedeuten:
R¹ C₁-C₄-Alkyl; und/oder
R⁵ Wasserstoff; und/oder
R² Wasserstoff, Halogen, C₁-C₄-Alkyl, C₁-C₉-Alkyloxy oder Amino.

Ferner sind bevorzugt Verbindungen der Formel 1, in denen
- R³: Wasserstoff, C₁-C₄-Alkyl, C₆-C₁₀-Aryl-C₁-C₄-alkyloxy, das gegebenenfalls im Arylteil durch Halogen substituiert sein kann oder NR⁶-A-R⁷ mit
R⁶ = Wasserstoff oder Benzyl,
A = einfacher Bindung und
R⁷ = C₆-C₁₀-Aryl-C₁-C₄-alkyl, das durch Halogen, CF₃, Cyano, Phenyl-C₁-C₄-alkyloxy, CF₃-Phenoxy, C₅-C₈-Cycloalkyl oder Fluorsulfonyloxy substituiert sein kann;
   C₁-C₁₂-Alkyl, das durch C₁-C₄-Alkyloxy, Phenyl, CF₃ oder Phenyl-C₁-C₄-alkyloxy substituiert sein kann;
   C₂-C₁₂-Alkenyl oder der Gruppe Het-(CH₂)ᵣ-, mit r = 0 oder 1, und Het = gesättigter oder ungesättigter 5-7-gliedriger Heterocyclus, der benzoanelliert und mit C₁-C₄-Alkyl oder Halogen substituiert sein kann, bedeutet.

Außerdem sind Verbindungen der Formel 1 bevorzugt, worin bedeuten:
R⁴ Wasserstoff, 2-Oxo-pyrrolidin-1-yl, 2,5-Dimethylpyrrol-1-yl oder C₆-C₁₀-Aryl-C₁-C₄-alkyloxy, das durch Halogen substituiert sein kann, und/oder

Verbindungen der Formel 1, worin bedeuten:
R⁴ = NR⁶-A-R⁷, mit
R⁶ = Wasserstoff oder Methyl,
A = einfache Bindung und
R⁷ = Wasserstoff;
   C₁-C₁₂-Alkyl, das ein- oder zweifach durch Halogen substituiert sein kann; C₂-C₁₈-Alkenyl, das ein- oder zweifach durch C₁-C₄-Alkyl oder C₁-C₄-Alkyloxycarbonyl substituiert sein kann;
   C₆-C₁₀-Aryl-C₁-C₄-alkyl, das durch Halogen, C₁-C₆-Alkyloxy, CF₃, Cyano, C₅-C₆-Cycloalkyl, C₁-C₄-Alkyloxycarbonyl, C₆-C₁₀-Aryl-C₁-C₄-alkyl, C₆-C₁₀-Aryl-C₁-C₄-alkyloxy substituiert sein kann, wobei Aryl durch Halogen oder CF₃ nochmals substituiert sein kann;
   C₅-C₈-Cycloalkyl-C₁-C₄-alkyl;
   oder der Gruppe Het-(CH₂)ᵣ-,
mit r = 1, 2 oder 3 und Het = gesättigter oder ungesättigter 5-7-gliedriger Heterocyclus, der mit Halogen, C₁-C₄-Alkyloxy oder C₁-C₄-Alkyloxycarbonyl substituiert sein kann, und/oder

Verbindungen der Formel 1, worin bedeuten:
R⁴ = NR⁶-A-R⁷, mit
R⁶ = Wasserstoff,
A = -CO- und
R⁷ = C₁-C₁₈-Alkyl, das durch Halogen, Phenyl, Phenoxy, Phenylcarbonyl oder C₁-C₄-Alkyloxycarbonyl substituiert sein kann, wobei Phenoxy wiederum durch Methyl, Halogen oder Methylmercapto substituiert sein kann;
   C₂-C₁₈-Alkenyl, das durch C₆-C₁₀-Aryl substituiert sein kann;
   C₆-C₁₀-Aryl, das durch Halogen, C₁-C₈-Alkyl, Phenyl-C₁-C₄-alkyl, CF₃, OCF₃, Fluorsulfonyl, C₁-C₄-Alkyloxycarbonyl, Phenoxy substituiert sein kann, wobei Aryl wiederum durch C₁-C₄-Alkyloxy substituiert sein kann;
   C₆-C₁₀-Aryl-C₁-C₄-alkyl, wobei Alkyl durch Methoxy oder CF₃ substituiert sein kann und Aryl durch Halogen;
   oder der Gruppe Het-(CH₂)ᵣ-,
   mit r = 0 und Het = gesättigter oder ungesättigter 5-7-gliedriger Heterocyclus, der benzoanelliert und mit C₁-C₄-Alkyl, Halogen, C₁-C₄-Alkyloxy, Halogenphenyl oder Halogen-benzylmercapto substituiert sein kann, wobei benzoanelliertes Aryl wiederum durch Halogen oder Methoxy substituiert sein kann, und/oder

Verbindungen der Formel 1, worin bedeuten:
R⁴ = NR⁶-A-R⁷, mit
R⁶ = Wasserstoff,
A = -CO₂- und
R⁷ = C₁-C₁₈-Alkyl, das durch CF₃ oder Phenyl substituiert ist; C₆-C₁₀-Aryl;
   C₆-C₁₀-Aryl-C₁-C₄-alkyl, das durch C₁-C₄-Alkyl, Halogen, CF₃, oder OCF₃,
   Benzyloxy oder Phenyl substituiert ist;
   oder der Gruppe Het-(CH₂)ᵣ-,
   mit r = 0 oder 1 und Het = gesättigter oder ungesättigter 5-7-gliedriger Heterocyclus, der benzoanelliert und mit C₁-C₄-Alkyl oder Benzyl substituiert sein kann, und/oder
R⁴ = NR⁶-A-R⁷, mit
R⁶ = Wasserstoff,
A = -SO₂- und
R⁷ = C₁-C₆-Alkyl, das mit CF₃ substituiert sein kann;
   C₂-C₄-Alkenyl, das durch Phenyl substituiert sein kann;
   C₆-C₁₀-Aryl, das durch C₁-C₆-Alkyl, Halogen, C₁-C₄-Alkyloxy oder Benzyl substituiert sein kann ;
   Diphenyl-C₁-C₄-alkyl, substituiert mit Halogen;
   oder der Gruppe Het-(CH₂)ᵣ-,
   mit r = 0 und Het = gesättigter oder ungesättigter 5-7-gliedriger Heterocyclus, und/oder

Verbindungen der Formel 1, worin bedeuten:
R⁴ = NR⁶-A-R⁷, mit
R⁶ = Wasserstoff,
A = -CO-NH- und
R⁷ = C₁-C₁₀-Alkyl, das durch C₁-C₄-Alkyloxycarbonyl, N(C₁-C₄-Alkyl)₂ oder Phenyl substituiert sein kann , das wiederum durch Halogen oder Aminosulfonyl substituiert sein kann;
   C₆-C₁₀-Aryl, das durch C₁-C₆-Alkyl, C₁-C₆-Alkyloxy, C₁-C₆-Alkyloxycarbonyl, Phenoxy, OCF₃, Benzyl oder Pyridyl, wobei Alkyl nochmals durch C₁-C₄-Alkyloxycarbonyl oder Carboxy substituiert sein kann;
   C₅-C₈-Cycloalkyl, das durch Hydroxy substituiert sein kann oder Indanyl; oder der Gruppe Het-(CH₂)ᵣ-,
   mit r = 0 oder 1 und Het = gesättigter oder ungesättigter 5-7-gliedriger Heterocyclus, der mit Benzyl substituiert sein kann.

Besonders bevorzugt sind Verbindungen der Formel 1, worin R¹ Methyl bedeutet.

Ganz besonders bevorzugt ist die Gruppe der in den Beispielen 21, 22, 27, 28, 30 bis 34, 36 bis 42, 53, 54, 58, 60, 62, 65, 69, 71, 74, 92, 97, 107, 116, 128, 130, 136, 139, 142, 152, 166 und 171 genannten Verbindungen der Formel 1.

Die erfindungsgemäßen Verbindungen der allgemeinen Formel 1 besitzen eine überraschende hemmende Wirkung an der hormonsensitiven Lipase, HSL, einem allosterischen Enzym in Adipozyten, das durch Insulin gehemmt wird und für den Abbau von Fetten in Fettzellen und damit für die Überführung von Fettbestandteilen in die Blutbahn verantwortlich ist. Eine Hemmung dieses Enzyms entspricht also einer insulinähnlichen Wirkung der erfindungsgemäßen Verbindungen, die letztlich zu einer Verminderung von freien Fettsäuren im Blut und von Blutzucker führt. Sie können also eingesetzt werden bei Entgleisungen des Stoffwechsels wie zum Beispiel beim nicht insulinabhängigen Diabetes Mellitus, beim diabetischen Syndrom und bei direkter Schädigung des Pankreas.

Die Herstellung der erfindungsgemäßen Verbindungen der allgemeinen Formel 1 kann nach an und für sich bekannten Methoden auf verschiedenen Wegen erfolgen.

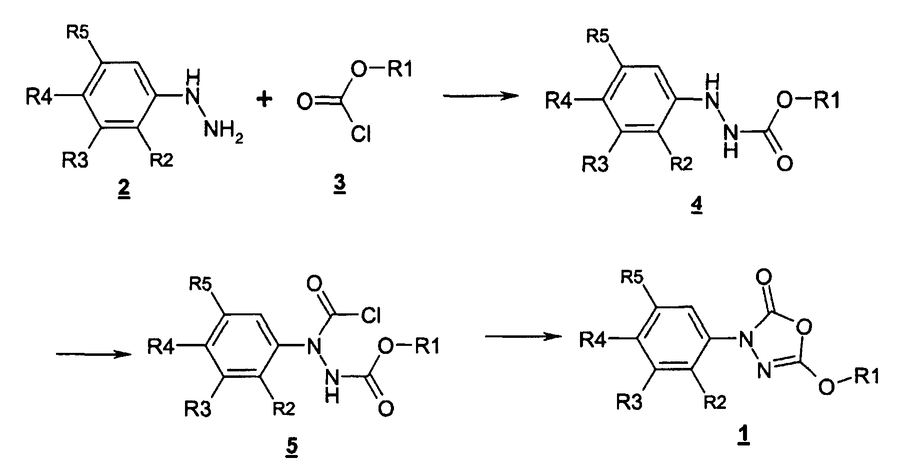

Beispielsweise kann die Herstellung der substituierten 3-Phenyl-5-alkoxi-1,3,4-oxdiazol-2-one der allgemeinen Formel 1 erfolgen, durch Umsetzung von Hydrazinen der allgemeinen Formel 2 mit Chlorameisensäureestern der Formel 3 oder anderen reaktiven Kohlensäureesterderivaten, worin R¹, R², R³, R⁴ und R⁵ wie oben definiert sind, zu den Verbindungen der Formel 4, welche mit Phosgen, Carbonyldiimidazol, Diphosgen oder Triphosgen acyliert, zyklisiert und gegebenenfalls durch weitere chemische Modifikation der Reste R²-R⁵, wie beispielsweise durch Reduktion von Nitro- zu Aminoresten nach bekannten Verfahren, und anschließende Acylierung oder Alkylierung, zu den Verbindungen der Formel 1 umgesetzt werden. Da bei diesen Reaktionen in der Regel Säuren freigesetzt werden, empfiehlt es sich zur Beschleunigung Basen wie Pyridin, Triethylamin, Natronlauge oder Alkalicarbonate zuzusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, nHeptan, Dioxan, Diethylether zu Einsatz.

Die Hydrazine der Formel 2 lassen sich nach bekannten Methoden z.B. durch Diazotierung der entsprechenden Aniline und 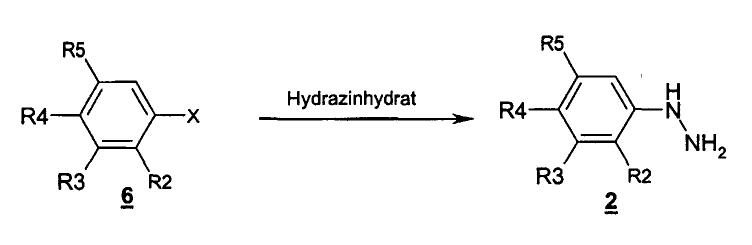 nachfolgender Reduktion nach bekannten Methoden oder durch nukleophile Substitution von geeignet substituierten Phenylderivaten 6 (X= F, Cl, Br, I, OSO₂CF₃) mit Hydrazinhydrat herstellen. Solche geeignete Phenylderivate können Nitro substituierte Halogenbenzole, vorzugsweise Fluor- und Chlor-nitrobenzole sein, aus denen sich an geeigneter Stelle im Syntheseweg durch Reduktion und Umsetzung mit Acylierungs- oder Alkylierungsmitteln ,wie beispielweise Säurechloriden, Anhydriden, Isocyanaten, Chlorameisensäureestern ,Sulfonsäurechloriden oder Alkyl- und Arylalkyl-halogeniden, oder durch reduktive Alkylierung mit Aldehyden nach bekannten Methoden die erfindungsgemäßen Verbindungen herstellen lassen.

Die Wirkung der erfindungsgemäßen Verbindungen der Formel 1 wurde an folgendem Enzymtestsytem geprüft:

### Enzympräparation:

### Präparation der partiell gereinigten HSL:

Isolierte Rattenfettzellen werden aus Nebenhodenfettgewebe von nicht-behandelten männlichen Ratten (Wistar, 220-250 g) durch Kollagenasebehandlung gemäß publizierter Verfahren gewonnen (z.B. S. Nilsson et al., Anal. Biochem. 158, 1986, 399 - 407; G. Fredrikson et al., J. Biol. Chem. 256, 1981, 6311 - 6320; H. Tomquist et al., J. Biol. Chem. 251, 1976, 813 - 819). Die Fettzellen aus 10 Ratten werden dreimal durch Flotation mit jeweils 50 ml Homogenisationspuffer (25 ml Tris/HCl, pH 7.4, 0.25 M Sucrose, 1 mM EDTA, 1 mM DTT, 10 µg/ml Leupeptin, 10 µg/ml Antipain, 20 µg/ml Pepstatin) gewaschen und schließlich in 10 ml Homogenisationspuffer aufgenommen. Die Fettzellen werden im Teflon-in-Glas Homogenisator (Braun-Melsungen) durch 10 Hübe bei 1500 rpm und 15°C homogenisiert. Das Homogenisat wird zentrifugiert (Sorvall SM24-Röhrchen, 5000 rpm, 10 min, 4°C). Der Unterstand zwischen der oben liegenden Fettschicht und dem Pellet wird abgenommen und die Zentrifugation wiederholt. Der daraus resultierende Unterstand wird erneut zentrifugiert (Sorvall SM24-Röhrchen, 20000 rpm, 45 min, 4°C). Der Unterstand wird abgenommen und mit 1 g Heparin-Sepharose (Pharmacia-Biotech, CL-6B, 5 x gewaschen mit 25 mM Tris/HCl, pH 7.4, 150 mM NaCl) versetzt. Nach Inkubation für 60 min bei 4°C (in Intervallen von 15 min aufschütteln) wird der Ansatz zentrifugiert (Sorvall SM24-Röhrchen, 3000 rpm, 10 min, 4°C). Der Überstand wird durch Zugabe von Eisessig auf pH 5.2 gebracht und 30 min bei 4°C inkubiert. Die Präzipitate werden durch Zentrifugation (Sorvall SS34, 12000 rpm, 10 min, 4°C) gesammelt und in 2.5 ml 20 mM Tris/HCl, pH 7.0, 1 mM EDTA, 65 mM NaCl, 13 % sucrose, 1 mM DTT, 10 µg/ml Leupeptin/Pepstatin/Antipain suspendiert. Die Suspension wird über Nacht bei 4°C gegen 25 mM Tris/HCl, pH 7.4, 50 % Glyzerin, 1 mM DTT, 10 µg/ml Leupeptin, Pepstatin, Antipain dialysiert und dann auf eine Hydroxiapatit-Säule aufgetragen (0.1 g pro 1 ml Suspension, äquilibriert mit 10 mM Kaliumphosphat, pH 7.0, 30 % Glyzerin, 1 mM DTT). Die Säule wird mit vier Volumina Äquilibrierungspuffer bei einer Flußrate von 20 bis 30 ml/h gewaschen. Die HSL wird mit einem Volumen Äquilibrierungspuffer, der 0.5 M Kaliumphosphat enthält, eluiert, sodann dialysiert (s.o.) und 5- bis 10-fach konzentriert durch Ultrafiltration (Amicon Diaflo PM 10 Filter) bei 4°C. Die partiell gereinigte HSL kann 4 bis 6 Wochen bei -70°C aufbewahrt werden.

### Assay:

Für die Herstellung des Substrats werden 25-50 µCi [³H]Trioleoylglycerin (in Toluol), 6.8 µMol unmarkiertes Trioleoylglycerin und 0.6 mg Phospholipide (Phosphatidylcholin/Phosphatidylinositol 3:1 w/v) gemischt, über N₂ getrocknet und dann in 2 ml 0.1 M KPᵢ (pH 7.0) durch Ultraschallbehandlung (Branson 250, Mikrospitze, Einstellung 1-2, 2 x 1 min im 1-min Intervall) aufgenommen. Nach Zugabe von 1 ml KPᵢ und erneuter Ultraschallbehandlung (4 x 30 sec auf Eis in 30-sec Intervallen) wird 1 ml 20% BSA (in KPᵢ) hinzugefügt (Endkonzentration Trioleoylglyzerin 1.7 mM). Für die Reaktion werden 100 µl Substratlösung zu 100 µl HSL-Lösung (HSL präpariert wie oben, verdünnt in 20 mM KPᵢ, pH 7.0, 1 mM EDTA, 1 mM DTT, 0.02% BSA, 20 µg/ml Pepstatin, 10 µg/ml Leupeptin) pipettiert und für 30 min bei 37°C inkubiert. Nach Zugabe von 3.25 ml Methanol/Chlorform/Heptan (10:9:7) und von 1.05 ml 0.1 M K₂CO₃, 0.1 M Borsäure (pH 10.5) wird gut gemischt und schließlich zentrifugiert (800 x g, 20 min). Nach Phasentrennung wird ein Äquivalent der oberen Phase (1 ml) abgenommen und die Radioaktivität durch Flüssigszintillationsmessung bestimmt.

### Auswertung:

Substanzen werden üblicherweise in vier unabhängigen Ansätzen geprüft. Die Hemmung der enzymatischen Aktivität der HSL durch eine Testsubstanz wird durch den Vergleich mit einer nicht-gehemmten Kontrollreaktion bestimmt. Die Berechnung des IC₅₀-Wertes erfolgt über eine Hemmkurve mit mind. 10 Konzentrationen der Testsubstanz. Für die Analyse der Daten wird das Softwarepaket GRAPHIT, Elsevier-BIOSOFT , benutzt.

In diesem Test zeigten die Verbindungen die folgende Wirkung:

| Verbindung Beispiel Nr. | IC₅₀ (µM) |
|---|---|
| 21 | 10 |
| 22 | 1 |
| 27 | 10 |
| 28 | 6 |
| 30 | 1 |
| 31 | 10 |
| 32 | 3 |
| 33 | 0,2 |
| 34 | 1 |
| 36 | 10 |
| 37 | 1 |
| 38 | 1 |
| 39 | 1 |
| 40 | 10 |
| 41 | 0,1 |
| 42 | 1 |
| 53 | 1 |
| 54 | 1 |
| 58 | 0,8 |
| 60 | 0,2 |
| 62 | 0,3 |
| 65 | 1 |
| 69 | 0,03 |
| 71 | 0,02 |
| 74 | 0,04 |
| 92 | 0,25 |
| 97 | 0,03 |
| 107 | 0,12 |
| 116 | 0,1 |
| 128 | 0,6 |
| 130 | 0,5 |
| 136 | 0,5 |
| 139 | 0,4 |
| 142 | 0,2 |
| 152 | 0,2 |
| 166 | 0,2 |
| 171 | 0,6 |

Die nachfolgenden Beispiele erläutern die Herstellungsmethoden näher, ohne sie einzuschränken.

### Beispiele:

### Beispiel 1:

### 3-Methyl-4-nitro-phenylhydrazin

Zur Lösung von 15,9 g 2-Methyl-4-fluor-nitrobenzol in 10 ml N-Methylpyrrolidon werden langsam bei Raumtemeratur 5 g Hydrazinhydrat zugetropft und die Mischung 4 Stunden auf 65 °C unter Umrühren erwärmt. Durch Zugabe von 70 ml Wasser wird das Produkt gefällt, abgesaugt und aus Isopropanol umkristallisiert.
Ausbeute:13,3 g Fp.: 138°C

In analoger Weise wurden die folgenden Beispiele hergestellt:

### Beispiel 2:

### 3-Fluor-4-nitro-phenylhydrazin

Fp.: 130°C

### Beispiel 3:

### 2-Chlor-4-nitro-phenylhydrazin

Fp.: 144°C

### Beispiel 4:

### 2-Methyl-4-nitro-phenylhydrazin

Fp.: 135°C

### Beispiel 5:

### 3-(4-Fluorbenzyloxi)-2-nitro-phenylhydrazin

Fp.: 164°C

Die Ausgangsverbindung, 2-Fluor-4-(4-fluorbenzyloxi)-nitrobenzol (Fp.: 99°C) wurde durch Alkylierung von 3-Fluor-4-nitro-phenol mit 4-Fluorbenzylchlorid in DMF in Gegenwart von Pottasche hergestellt.

### Beispiel 6:

### 3-(4-Fluorbenzyloxi)-4-nitro-phenylhydrazin (Zwischenprodukt)

Fp.: 145°C

### Beispiel 7:

### 4-(4-Chlorphenoxi)-3-nitro-anilin

Zur Lösung von 1,29 g 4-Chlorphenol in 8 ml DMF werden 1,4 g Pottasche gefügt und nach 30 minütigem Umrühren werden 1,6 g 4-Fluor-3-nitro-anilin zugegeben und die Mischung 3 Stunden bei 100°C gerührt. Nach dem Erkalten werden 80 ml Wasser zugefügt und der Niederschlag nach kurzem Verrühren abgesaugt und im Vakuum bei 40°C getrocknet.
Ausbeute: 2,0 g; Fp.: 101°C

### Beispiel 8:

### 4-(4-Chlorphenoxi)-3-nitro-phenylhydrazin

Zur auf 0°C abgekühlten, gerührten Mischung bestehend aus 1,9 g 4-(4-Chlorphenoxi)-3-nitro-anilin, 25 ml konz. Salzsäure und 25 ml Ethanol wird die Lösung von 0,52 g Natriumnitrit in 5 ml Wasser zugetropft und die Mischung bei -0°C 60 Min nachgerührt und anschließend zur Suspension von 8,5 g Zinndichloriddihydrat in 8 ml konz. HCl zugetropft. Der Niederschlag wird abgesaugt, mit Wasser gewaschen, unter Stickstoff in 200 ml Wasser aufgeschlämmt und mit 100 ml 30%ige Natronlauge bei 10-15°C zersetzt. Das sich bildende Öl wird mit Essigester ausgeschüttelt, mit Wasser gewaschen, die organische Phase mit Natriumsulfat getrocknet. Dann wird das Produkt mit isopropanolischer HCl gefällt, abgesaugt und im Vakuum getrocknet.
Ausbeute: 1,1 g; Fp.: 221°C

### Beispiel 9:

### N'-(4-Nitro-2-methyl-phenyl)-hydrazino-ameisensäuremethylester

Zur Mischung bestehend aus 0,84 g 2-Methyl-4-nitro-phenylhydrazin, 15 ml NMP und 2 ml Pyridin wurden vorsichtig unter Eiskühlung 0,43 ml Chlorameisensäuremethylester zugetropft und dann unter langsamen Erwärmen auf RT 2 Stunden gerührt. Nach Verdünnen mit 50 ml Wasser wurde die Mischung über Nacht gerührt und der Feststoff im Vakuum bei 40 °C getrocknet.
Ausbeute: 0,81 g; Fp.:153°C

Die folgenden Beispiele wurden in ähnlicher Weise hergestellt:

### Beispiel 10:

### N'-(4-Nitro-phenyl)-hydrazino-ameisensäuremethylester (Zwischenprodukt)

Fp.: 179°C

### Beispiel 11:

### N'-(3-Fluor-4-nitro-phenyl)-hydrazino-ameisensäuremethylester

Fp.: 127,4°C

### Beispiel 12:

### N'-(3-Methyl-4-nitro-phenyl)-hydrazino-ameisensäuremethylester

Fp.: 159°C

### Beispiel 13:

### N'-(2-Chlor-4-nitro-phenyl)-hydrazino-ameisensäuremethylester

Fp.: 156°C

### Beispiel 14:

### N'-(3-(4-Fluorbenzyloxi)-4-nitro-phenyl)-hydrazino-ameisensäuremethylester (Zwischenprodukt) Fp.: 166°C

### Beispiel 15:

### N'-(3-(4-Fluorbenzyloxi)-2-nitro-phenyl)-hydrazino-ameisensäuremethylester

Fp.: 193°C

### Beispiel 16:

### N'-(4-(4-Chlorphenoxi)-3-nitro-phenyl)-hydrazino-ameisensäuremethylester

Fp.: 147°C

### Beispiel 17:

### N'-(3-Piperidino-4-nitro-phenyl)-hydrazino-ameisensäuremethylester (-)

Fp.: 131°C

Diese Verbindung und die Verbindung des Beispiels 18 wurden durch Umsetzung von N'-(3-Fluor-4-nitro-phenyl)-hydrazino-ameisensäuremethylester mit Piperidin bzw. N-Benzyl-piperazin in NMP bei 80°C hergestellt.

### Beispiel 18:

### N'-(3-(N-Benzyl-piperazino)-4-nitro-phenyl)-hydrazino-ameisensäuremethylester

Fp.: 156°C

### Beispiel 19:

### 5-Methoxi-3-(4-nitro-phenyl)-3-H-(1,3,4)oxdiazol-2-on

2,5 g N'-(4-Nitro-phenyl)-hydrazino-ameisensäuremethylester und 5ml Pyridin wurden in 15 ml Methylenchlorid aufgenommen und unter Umrühren und Eiskühlung mit 3 ml einer 20%igen toluolischen Phosgenlösung tropfenweise versetzt. Diese Mischung stand über Nacht bei Raumtemperatur und wurde mit weiteren 10 ml Methylenchlorid verdünnt und dann 3 mal mit Wasser gewaschen. Nach dem Trocknen über Natriumsulfat wurde die Mischung im Vakuum eingeengt und das Produkt durch Säulenchromatografie (Kieselgel, Lösungsmittel:
Methanol:Methylenchlorid = 2 : 98) gereinigt und aus Isopropanol umkristallisiert.
Ausbeute:1,5g Fp.: 151°C

Die folgenden Beispiele wurden analog Beispiel 4 hergestellt:

### Beispiel 20:

### 5-Methoxi-3-(3-methyl-4-nitro-phenyl)-3-H-(1,3,4)oxdiazol-2-on

Fp.: 112°C

### Beispiel 21:

### 5-Methoxi-3-(4-(4-chlorphenoxi-3-nitro-phenyl)-3-H-(1,3,4)oxdiazol-2-on

Fp.: Öl

### Beispiel 22:

### 5-Methoxi-3-(3-(4-fluorbenzyloxi)-2-nitro-phenyl)-3-H-(1,3,4)oxdiazol-2-on

Fp.: 99°C

### Beispiel 23:

### 5-Methoxi-3-(2-methyl-4-nitro-phenyl)-3-H-(1,3,4)oxdiazol-2-on

Fp.: 111°C

### Beispiel 24:

### 5-Methoxi-3-(3-(4-fluorbenzyloxi)-4-nitro-phenyl)-3-H-(1,3,4)oxdiazol-2-on

Fp.: 137°C

### Beispiel 25:

### 5-Methoxi-3-(4-amino-phenyl)-3-H-(1,3,4)oxdiazol-2-on

Die Mischung bestehend aus 1,4 g 5-Methoxi-3-(4-nitro-phenyl)-3-H-(1,3,4)oxdiazol-2-on, 0,5 g Pd/C und 20ml Methanol wird unter Normaldruck bei Raumtemperatur bis zur Aufnahme der berechneten Wasserstoffmenge hydriert. Dann wird vom Katalysator abfiltriert und die Lösung im Vakuum eingeengt. Der verbleibende halbfeste Rückstand wird mit Isopropanol verrührt und abgesaugt.
Ausbeute: 0,75g; Fp.: 85°C

### Beispiel 26:

### 5-Methoxi-3-(2-amino-4-(4-fluorbenzyloxi)-phenyl)-3-H-(1,3,4)oxdiazol-2-on

Fp.: Öl

### Beispiel 27:

### 5-Methoxi-3-(3-amino-4-(4-chlorphenoxi)-phenyl)-3-H-(1,3,4)oxdiazol-2-on

Fp.: 133°C

### Beispiel 28:

### 5-Methoxi-3-(4-amino-3-methyl-phenyl)-3-H-(1,3,4)oxdiazol-2-on

Fp.: 114°C

### Beispiel 29:

### 5-Methoxi-3-(4-amino-3-(4-fluorbenzyloxi)-phenyl)-3-H-(1,3,4)oxdiazol-2-on

Fp.: 195°C

### Beispiel 30:

### 5-Methoxi-3-(4-(4-chlorphenylacetylamino)-phenyl)-3-H-(1,3,4)oxdiazol-2-on

Zur eisgekühlten Mischung bestehend aus 200 mg 5-Methoxi-3-(4-amino-phenyl)-3-H-(1,3,4)oxdiazol-2-on, 20 ml Methylenchlorid und 0,1 ml Pyridin werden 201 mg 4-Chlorphenylessigsäurechlorid getropft und der Ansatz 5 Stunden bei Raumtemperatur gerührt. Die flüchtigen Anteile werden im Vakuum entfernt und der Rückstand mit Wasser verrührt und der Feststoff abgesaugt und bei 40°C im Vakuum getrocknet.
Ausbeute: 318 mg; Fp.:161°C

In analoger Weise wurden die folgenden Beispiele hergestellt:

### Beispiel 31:

### 5-Methoxi-3-(4-(4-chlorphenylacetylamino)-3-methyl-phenyl)-3-H-(1,3,4)oxdiazol-2-on

Fp.: 190°C

### Beispiel 32:

### 5-Methoxi-3-(4-octanoylamino-3-methyl-phenyl)-3-H-(1,3,4)oxdiazol-2-on

Fp.: 110°C

### Beispiel 33:

### 5-Methoxi-3-(4-(4-heptyl-benzoylamino)-phenyl)-3-H-(1,3,4)oxdiazol-2-on

Fp.: 155°C

### Beispiel 34:

### 5-Methoxi-3-(4-(4-butyl-phenyl-sulfonylamino)-phenyl)-3-H-(1,3,4)oxdiazol-2-on

Fp.: 135°C

### Beispiel 35:

### 5-Methoxi-3-(4-(4-chlorbutanoylamino)-3-methyl-phenyl)-3-H-(1,3,4)oxdiazol-2-on

Fp.: 137°C

### Beispiel 36:

### 5-Methoxi-3-(4-pivaloylamino-3-methyl-phenyl)-3-H-(1,3,4)oxdiazol-2-on

Fp.: 157°C

### Beispiel 37:

### 5-Methoxi-3-(4-(4-chlorphenylsulfonylamino)-3-methyl-phenyl)-3-H-(1,3,4)oxdiazol-2-on

Fp.: 147°C

### Beispiel 38:

### 5-Methoxi-3-(4-(1-naphthylsulfonylamino)-3-methyl-phenyl)-3-H-(1,3,4)oxdiazol-2-on

Fp.: 123°C

### Beispiel 39:

### 5-Methoxi-3-(4-(2-phenylethenylsulfonylamino)-3-methyl-phenyl)-3-H-(1,3,4)oxdiazol-2-on

Fp.: 129°C

### Beispiel 40:

### 5-Methoxi-3-(4-(2,2,2-trifluorethyl-sulfonylamino)-3-methyl-phenyl)-3-H-(1,3,4)oxdiazol-2-on

Fp.: 151°C

### Beispiel 41:

### 5-Methoxi-3-(4-(benzyloxicarbonylamino)-3-methyl-phenyl)-3-H-(1,3,4)oxdiazol-2-on

Fp.: 115°C

### Beispiel 42:

### 5-Methoxi-3-(4-(3,4-dichlorphenylamino-carbonylamino)-3-methyl-phenyl)-3-H-(1,3,4)oxdiazol-2-on

Fp.: 210°C

Diese Verbindung wurde durch Umsetzung von 5-Methoxi-3-(4-amino-3-methylphenyl)-3-H-(1,3,4)oxdiazol-2-on mit äquimolaren Mengen 3,4-Dichlorphenylisocyanat in Toluol bei 50°C erhalten.

### Beispiel 43:

### 5-Methoxi-3-(4-(4-chlorphenylsulfonylamino)-phenyl)-3-H-(1,3,4)oxdiazol-2-on

Fp.: 169°C

### Beispiel 44:

### 5-Methoxi-3-(4-(2-chlorphenylsulfonylamino)-phenyl)-3-H-(1,3,4)oxdiazol-2-on

Fp.: 171°C

### Beispiel 45:

### 5-Methoxi-3-(4-(3-chlorphenylsulfonylamino)-phenyl)-3-H-(1,3,4)oxdiazol-2-on

Fp.: 141°C

### Beispiel 46:

### 5-Methoxi-3-(4-(4-chlorphenylacetyl-amino)-3-(4-fluorbenzyloxi)-phenyl)-3-H-(1,3,4)oxdiazol-2-on

Fp.: 167°C

### Beispiel 47:

### 5-Methoxi-3-(4-benzylsulfonylamino-phenyl)-3-H-(1,3,4)oxdiazol-2-on

Fp.: 153°C

### Beispiel 48:

### 5-Methoxi-3-(4-(-2-(4'-chlor-biphenyl)-ethyl)-sulfonylamino)-phenyl)-3-H-(1,3,4)oxdiazol-2-on

Fp.: 165°C

### Beispiel 49:

### 5-Methoxi-3-(4-isopropylsulfonylamino-phenyl)-3-H-(1,3,4)oxdiazol-2-on

Fp.: 190°C

### Beispiel 50:

### 5-Methoxi-3-(4-dimethylamino-3-methyl-phenyl)-3-H-(1,3,4)oxdiazol-2-on

Fp.: 71°C

Diese Verbindung wurde durch Umsetzung 5-Methoxi-3-(4-amino-3-methyl-phenyl)-3-H-(1,3,4)oxdiazol-2-on mit Paraformaldehyd/Ameisensäure in DMF bei Raumtemperatur erhalten und durch Säulenchromatografie (Kieselgel, Essigester:n-Heptan=1:1) gereinigt.

### Beispiel 51:

### 5-Methoxi-3-(4-(4-chlorbenzylamino)-3-methyl-phenyl)-3-H-(1,3,4)oxdiazol-2-on

Fp.: Öl

Diese Verbindung wurde durch Umsetzung 5-Methoxi-3-(4-amino-3-methyl-phenyl)-3-H-(1,3,4)oxdiazol-2-on mit 4-Chlorbenzaldehyd/Natriumborhydrid in Methanol/Methylenchlorid bei Raumtemperatur erhalten und durch Säulenchromatografie (Kieselgel, Essigester:n-Heptan=1:1) gereinigt.

### Beispiel 52:

### 5-Methoxi-3-(4-(2-oxo-pyrrolidin-1-yl)-3-methyl-phenyl)-3-H-(1,3,4)oxdiazol-2-on

Fp.: Öl

Diese Verbindung wurde durch Umsetzung von 5-Methoxi-3-(4-(4-chlorbutanoylamino)-3-methyl-phenyl)--3-H-(1,3,4)oxdiazol-2-on mit Natriumhydrid in Dioxan bei Raumtemperatur und Reinigung des Rohprodukts durch Säulenchromatografie (Kieselgel, Methylenchlorid:Methanol=98:2) hergestellt.

### Beispiel 53:

### 5-Methoxi-3-(4-(4-oxo-pent-2-en-2-yl-amino)-3-methyl-phenyl)-3-H-(1,3,4)oxdiazol-2-on

Fp.: 143°C

Diese Verbindung wurde durch Umsetzung 5-Methoxi-3-(4-amino-3-methyl-phenyl)-3-H-(1,3,4)oxdiazol-2-on mit äquimolaren Mengen Acetylaceton in Eisessig bei 80°C erhalten und durch Ausfällen durch Zugabe von Wasser und Filtration isoliert.

### Beispiel 54:

### 5-Methoxi-3-(4-(2,5-dimethyl-pyrrol-1-yl)-3-methyl-phenyl)-3-H-(1,3,4)oxdiazol-2-on

Fp.: Öl

Diese Verbindung wurde durch Umsetzung 5-Methoxi-3-(4-amino-3-methyl-phenyl)-3-H-(1,3,4)oxdiazol-2-on mit äquimolaren Mengen Acetonylaceton in Eisessig bei 80°C erhalten. Die Aufarbeitung erfolgte durch Verdünnung mit Wasser, Ausschütteln mit Essigester und Säulenchromatografie (Kieselgel, Methylenchlorid) des nach dem Einengen der getrockneten organische Phase erhaltenen Rohproduktes.

### Beispiel 55:

### 5-Methoxi-3-(3-(4-fluorbenzyloxi)-4-methylamino-phenyl)-3-H-(1,3,4)oxdiazol-2-on

Fp.: 98°C

Diese Verbindung wurde als Nebenprodukt bei der Hydrierung von 5-Methoxi-3-(3-(4-fluorbenzyloxi)-4-nitro-phenyl)-3-H-(1,3,4)oxdiazol-2-on mit Platindioxid als Katalysator in Methanol bei Raumtemperatur unter Normaldruck und nach Abfiltrieren des Katalysators , Einengen der Reaktionsmischung und Säulenchromatografie (Kieselgel, Methylenchlorid) erhalten.

Die Verbindungen der Beispiele 56 - 199 wurden analog den vorstehenden Beispielen hergestellt.

### Beispiel 56:

### 5-Methoxi-3-(3-amino-phenyl)-3-H-(1,3,4)oxdiazol-2-on

Fp.: 95°C

### Beispiel 57:

### 5-Methoxi-3-(3-dibenzylamino-phenyl)-3-H-(1,3,4)oxdiazol-2-on

Fp.: 71 °C

### Beispiel 58:

### 5-Methoxi-3-(3-benzylamino-phenyl)-3-H-(1,3,4)oxdiazol-2-on

Fp.: Öl

### Beispiel 59:

### 5-Methoxi-3-(4-(pyrid-2-yl)-amino-carbonyl-amino-phenyl)-3-H-(1,3,4)oxdiazol-2-on

Fp.: 81 °C

### Beispiel 60:

### 5-Methoxi-3-(3-(4-fluorbenzyl-oxi)-4-benzyloxicarbonylamino-phenyl)-3-H-(1,3,4)oxdiazol-2-on

Fp.: Öl

### Beispiel 61:

### 5-Methoxi-3-(4-amino-2-methyl-phenyl)-3-H-(1,3,4)oxdiazol-2-on

Fp.: Öl

### Beispiel 62:

### 5-Methoxi-3-(3-methyl-4-(2-chlorbenzyloxicarbonylamino)-phenyl)-3-H-(1,3,4)oxdiazol-2-on

Fp.: 161 °C

### Beispiel 63:

### 5-Methoxi-3-(4-amino-2-chlor-phenyl)-3-H-(1,3,4)oxdiazol-2-on

Fp.: 126 °C

### Beispiel 64:

### 5-Methoxi-3-(2-chlor-4-nitro-phenyl)-3-H-(1,3,4)oxdiazol-2-on

Fp.: 92 °C

### Beispiel 65:

### 5-Methoxi-3-(2-methyl-4-benzyloxicarbonylamino-phenyl)-3-H-(1,3,4)oxdiazol-2-on

### Fp.: 112 °C

### Beispiel 66:

### 5-Methoxi-3-(2-methyl-4-(4-trifluormethoxibenzoylamino)-phenyl)-3-H-(1,3,4)oxdiazol-2-on

Fp.: 150 °C

### Beispiel 67:

### 5-Methoxi-3-(2-chlor-4-benzyloxicarbonylamino-phenyl)-3-H-(1,3,4)oxdiazol-2-on

Fp.: 150 °C

### Beispiel 68:

### 5-Methoxi-3-(3-fluor-4-nitro-phenyl)-3-H-(1,3,4)oxdiazol-2-on

Fp.: 127 °C

### Beispiel 69:

### 5-Methoxi-3-(4-(4-t-butyl-benzoylamino)-phenyl)-3-H-(1,3,4)oxdiazol-2-on

Fp.: 173 °C

### Beispiel 70:

### 5-Methoxi-3-(4-(4-chlor-benzyloxicarbonylamino)-phenyl)-3-H-(1,3,4)oxdiazol-2-on

Fp.: 177 °C

### Beispiel 71:

### 5-Methoxi-3-(2-chlor-4-(4-heptylbenzoylamino)-phenyl)-3-H-(1,3,4)oxdiazol-2-on

Fp.: 135 °C

### Beispiel 72:

### 5-Methoxi-3-(4-(3,4-dichlorbenzoylamino)-phenyl)-3-H-(1,3,4)oxdiazol-2-on

Fp.: 200 °C

### Beispiel 73:

### 5-Methoxi-3-(4-(2-(4-chlorphenoxi)-2-methyl-propionylamino)-phenyl)-3-H-(1,3,4)oxdiazol-2-on

Fp.: 153 °C

### Beispiel 74:

### 5-Ethoxi-3-(3-methyl-4-benzyloxicarbonylamino-phenyl)-3-H-(1,3,4)oxdiazol-2-on

Fp.: 94 °C

### Beispiel 75:

### 5-Isopropoxi-3-(3-methyl-4-benzyloxicarbonylamino-phenyl)-3-H-(1,3,4)oxdiazol-2-on

Fp.: 119°C

### Beispiel 76:

### 5-Isopropoxi-3-(3-methyl-4-butyloxicarbonylamino-phenyl)-3-H-(1,3,4)oxdiazol-2-on

Fp.: 114 °C

### Beispiel 77:

### 5-Isopropoxi-3-(3-methyl-4-(3-chlorphenylamino-carbonylamino)-phenyl)-3-H-(1,3,4)oxdiazol-2-on

Fp.: 201 °C

### Beispiel 78:

### 5-tert.Butoxi-3-(3-methyl-4-benzyloxicarbonylamino-phenyl)-3-H-(1,3,4)oxdiazol-2-on

Fp.: 113 °C

### Beispiel 79:

### 5-Methoxi-3-(3-methyl-4-phenoxicarbonylamino-phenyl)-3-H-(1,3,4)oxdiazol-2-on

Fp.: 145 °C

### Beispiel 80:

### 5-Methoxi-3-(3-methyl-4-(pyrid-3-yl-carbonylamino)-phenyl)-3-H-(1,3,4)oxdiazol-2-on

Fp.: Öl

### Beispiel 81:

### 5-Methoxi-3-(3-methyl-4-(indan-2-yl-aminocarbonylamino)-phenyl)-3-H-(1,3,4)oxdiazol-2-on

Fp.: 206 °C

### Beispiel 82:

### 5-Methoxi-3-(3-methyl-4-(pyrid-3-yl-methylaminocarbonylamino)-phenyl)-3-H-(1,3,4)oxdiazol-2-on

Fp.: 229 °C

### Beispiel 83:

### 5-Methoxi-3-(3-methyl-4-(pyrid-3-yl-methoxicarbonylamino)-phenyl)-3-H-(1,3,4)oxdiazol-2-on

Fp.: 232 °C

### Beispiel 84:

### 5-Methoxi-3-(3-fluor-4-benzyloxicarbonylamino-phenyl)-3-H-(1,3,4)oxdiazol-2-on

Fp.: Öl

### Beispiel 85:

### 5-Methoxi-3-(3-fluor-4-(4-trifluormethylbenzoylamino)-phenyl)-3-H-(1,3,4)oxdiazol-2-on

Fp.: Öl

### Beispiel 86:

### 5-Methoxi-3-(3-benzyloxi-4-(4-trifluormethylbenzoylamino)-phenyl)-3-H-(1,3,4)oxdiazol-2-on

Fp.: 159 °C

### Beispiel 87:

### 5-Methoxi-3-(3-fluor-4-(4-tert.butyl-benzoylamino)-phenyl)-3-H-(1,3,4)oxdiazol-2-on

Fp.: 144 °C

### Beispiel 88:

### 5-Methoxi-3-(3-methyl-4-(2,2,2-trifluorethoxicarbonylamino)-phenyl)-3-H-(1,3,4)oxdiazol-2-on

Fp.: 141 °C

### Beispiel 89:

### 5-Methoxi-3-(3-methyl-4-piperidinocarbonylamino-phenyl)-3-H-(1,3,4)oxdiazol-2-on

Fp.: 154 °C

### Beispiel 90:

### 5-Methoxi-3-(4-(6-methoxi-benzofuran-2-yl-carbonylamino)-phenyl)-3-H-(1,3,4)oxdiazol-2-on

Fp.: 191 °C

Weitere Beispiele, die nach den oben beschriebenen Verfahren hergestellt wurden und durch Massenspektroskopie (M+1 ) charakterisiert wurden:

| Beispiel Nr | Chemische Bezeichnung: | M+1 | Molgew. |
|---|---|---|---|
| 91 | N-[4-(5-Methoxi-2-oxo-[1,3,4]oxdiazol-3-yl)-phenyl]-3-methylbenzolsulfonamid | 362 | 361,4 |
| 92 | 3,4-Dimethoxi-N-[4-(5-methoxi-2-oxo-[1,3,4]oxdiazol-3-yl)-phenyl]benzolsulfonamid | 408 | 407,4 |
| 93 | Chinolin-8-sulfonsäure-[4-(5-methoxi-2-oxo-[1,3,4]oxdiazol-3-yl)-phenyl]-amid | 399 | 398,4 |
| 94 | N-[4-(5-Methoxi-2-oxo-[1,3,4]oxdiazol-3-yl)-phenyl]-5-nitroisophthalsäuremonomethylester | 415 | 414,3 |
| 95 | 3-(2-Chlor-phenyl)-5-methyl-isoxazole-4-carbonsäure [4-(5-methoxi-2-oxo-[1,3,4]oxdiazol-3-yl)-phenyl]-amid | 427 | 426,8 |
| 96 | 3,3,3-Trifluor-2-methoxi-N-[4-(5-methoxi-2-oxo-[1,3,4]oxdiazol-3-yl)-phenyl]-2-phenyl-propionamid | 424 | 423,3 |
| 97 | 2-Fluor-N-[4-(5-methoxi-2-oxo-[1,3,4]oxdiazol-3-yl)-phenyl]-benzamid | 330 | 329,3 |
| 98 | Tetradecansäure [4-(5-methoxi-2-oxo-[1,3,4]oxdiazol-3-yl)-phenyl]-amid | 418 | 417,5 |
| 99 | N-[4-(5-Methoxi-2-oxo-[1,3,4]oxdiazol-3-yl)-phenyl]-2-phenethylbenzamid | 416 | 415,4 |
| 100 | N-[4-(5-Methoxi-2-oxo-[1,3,4]oxdiazol-3-yl)-phenyl]-2-(4-methoxiphenoxy)-5-nitro-benzamid | 479 | 478,4 |
| 101 | 2-(4-Benzyloxy-phenyl)-N-[4-(5-methoxi-2-oxo-[1,3,4]oxdiazol-3-yl)-phenyl]-acetamid | 432 | 431,4 |
| 102 | N-[4-(5-Methoxi-2-oxo-[1,3,4]oxdiazol-3-yl)-phenyl]-3,3,3-triphenylpropionamid | 492 | 491,5 |
| 103 | N-[4-(5-Methoxi-2-oxo-[1,3,4]oxdiazol-3-yl)-phenyl]-3,5-bis-trifluormethyl-benzamid | 448 | 447,3 |
| 104 | 4-Cyano-N-[4-(5-methoxi-2-oxo-[1,3,4]oxdiazol-3-yl)-phenyl]-benzamid | 337 | 336,3 |
| 105 | Nonansäure [4-(5-methoxi-2-oxo-[1,3,4]oxdiazol-3-yl)-phenyl]-amid | 348 | 347,4 |
| 106 | 9-[4-(5-Methoxi-2-oxo-[1,3,4]oxdiazol-3-yl)-phenylcarbamoyl]-nonansäure-methylester | 406 | 405,4 |
| 107 | Undecansäure [4-(5-methoxi-2-oxo-[1,3,4]oxdiazol-3-yl)-phenyl]-amid | 376 | 375,5 |
| 108 | 4-[4-(5-Methoxi-2-oxo-[1,3,4]oxdiazol-3-yl)-phenylcarbamoyl]-benzolsulfonylfluorid | 394 | 393,3 |
| 109 | 11-Phenoxy-undecansäure [4-(5-methoxi-2-oxo-[1,3,4]oxdiazol-3-yl)-phenyl]-amid | 468 | 467,6 |
| 110 | N-[4-(5-Methoxi-2-oxo-[1,3,4]oxdiazol-3-yl)-phenyl]-2,3-diphenylpropionamid | 416 | 415,4 |
| 111 | 4-Chlor-N-[4-(5-methoxi-2-oxo-[1,3,4]oxdiazol-3-yl)-phenyl]-2-methylbenzamid | 360 | 359,8 |
| 112 | 6-Chlor-N-[4-(5-methoxi-2-oxo-[1,3,4]oxdiazol-3-yl)-phenyl]-nicotinamid | 347 | 346,7 |
| 113 | 5-Fluor-N-[4-(5-methoxi-2-oxo-[1,3,4]oxdiazol-3-yl)-phenyl]-2-methylbenzamid | 344 | 343,3 |
| 114 | N-[4-(5-Methoxi-2-oxo-[1,3,4]oxdiazol-3-yl)-phenyl]-2,4,6-trimethylbenzamid | 354 | 353,4 |
| 115 | N-[4-(5-Methoxi-2-oxo-[1,3,4]oxdiazol-3-yl)-phenyl]-3-naphthalen-2-yl-acrylamid | 388 | 387,4 |
| 116 | 5-Oxo-5-phenyl-pentansäure [4-(5-methoxi-2-oxo-[1,3,4]oxdiazol-3-yl)-phenyl]-amid | 382 | 381,4 |
| 117 | 3-(2,4-Dichlor-benzylsulfanyl)-thiophene-2-carbonsäure [4-(5-methoxi-2-oxo-[1,3,4]oxdiazol-3-yl)-phenyl]-amid | 509 | 508,4 |
| 118 | 2-Fluor-N-[4-(5-methoxi-2-oxo-[1,3,4]oxdiazol-3-yl)-phenyl]-4-trifluormethyl-benzamid | 398 | 397,3 |
| 119 | 1-Hexyl-3-[3-(5-methoxi-2-oxo-[1,3,4]oxdiazol-3-yl)-phenyl]-harnstoff | 335 | 334,4 |
| 120 | 1-(4-Bromo-phenyl)-3-[3-(5-methoxi-2-oxo-[1,3,4]oxdiazol-3-yl)-phenyl]-harnstoff | 406 | 405,2 |
| 121 | 1-[3-(5-Methoxi-2-oxo-[1,3,4]oxdiazol-3-yl)-phenyl]-3-(2-methoxiphenyl)-harnstoff | 357 | 356,3 |
| 122 | 2-[3-[3-(5-Methoxi-2-oxo-[1,3,4]oxdiazol-3-yl)-phenyl]-ureido]-3-phenyl-propionsäureethylester | 427 | 426,4 |
| 123 | 1-(2,6-Diisopropyl-phenyl)-3-[3-(5-methoxi-2-oxo-[1,3,4]oxdiazol-3-yl)-phenyl]-harnstoff | 411 | 410,5 |
| 124 | 1-[3-(5-Methoxi-2-oxo-[1,3,4]oxdiazol-3-yl)-phenyl]-3-octylharnstoff | 363 | 362,4 |
| 125 | 1-(4-Fluor-benzyl)-3-[3-(5-methoxi-2-oxo-[1,3,4]oxdiazol-3-yl)-phenyl]-harnstoff | 359 | 358,3 |
| 126 | 1-(2-Ethyl-phenyl)-3-[3-(5-methoxi-2-oxo-[1,3,4]oxdiazol-3-yl)-phenyl]-harnstoff | 355 | 354,4 |
| 127 | 6-[3-[3-(5-Methoxi-2-oxo-[1,3,4]oxdiazol-3-yl)-phenyl]-ureido]-hexansäure-ethylester | 393 | 392,4 |
| 128 | 1-(2,6-Dimethoxi-phenyl)-3-[3-(5-methoxi-2-oxo-[1,3,4]oxdiazol-3-yl)-phenyl]-harnstoff | 387 | 386,4 |
| 129 | 5-Methoxi-3-[4-[(thiophen-3-ylmethyl)-amino]-phenyl]-3H-[1,3,4]oxdiazol-2-on | 304 | 303,3 |
| 130 | 4-[[4-(5-Methoxi-2-oxo-[1,3,4]oxdiazol-3-yl)-phenylamino]-methyl]benzonitrile-trifluoracetat | 437 | 436,3 |
| 131 | 3-[4-(2-Brom-4,5-dimethoxi-benzylamino)-phenyl]-5-methoxi-3H-[1,3,4]oxdiazol-2-on | 437 | 436,3 |
| 132 | 3-[4-(3-Ethoxi-4-methoxi-benzylamino)-phenyl]-5-methoxi-3H-[1,3,4]oxdiazol-2-on-trifluoracetat | 486 | 485,4 |
| 133 | 4-[[4-(5-Methoxi-2-oxo-[1,3,4]oxdiazol-3-yl)-phenylamino]-methyl]-benzoesäuremethylester-trifluoracetat | 470 | 469,4 |
| 134 | Essigsäure-4-[[4-(5-methoxi-2-oxo-[1,3,4]oxdiazol-3-yl)-phenylamino]-methyl]-phenyl ester | 356 | 355,3 |
| 135 | 5-Methoxi-3-[4-(pentafluorphenylmethyl-amino)-phenyl]-3H-[1,3,4]oxdiazol-2-on | 388 | 387,3 |
| 136 | 3-[4-(4-Benzyloxy-benzylamino)-phenyl]-5-methoxi-3H-[1,3,4]oxdiazol-2-on-trifluoracetat | 518 | 517,5 |
| 137 | 3-[4-(3,3-Dichlor-nonylamino)-phenyl]-5-methoxi-3H-[1,3,4]oxdiazol-2-on-trifluoracetat | 517 | 516,3 |
| 138 | 2-[[4-(5-Methoxi-2-oxo-[1,3,4]oxdiazol-3-yl)-phenylamino]-methyl]-benzonitrile | 323 | 322,3 |
| 139 | 3-[4-(Cyclohexylmethyl-amino)-phenyl]-5-methoxi-3H-[1,3,4]oxdiazol-2-on | 304 | 303,4 |
| 140 | 5-Methoxi-3-[4-(2,3,5-trichlor-benzylamino)-phenyl]-3H-[1,3,4]oxdiazol-2-on-trifluoracetat | 515 | 514,7 |
| 141 | 3-[4-(5-Brom-2-fluor-benzylamino)-phenyl]-5-methoxi-3H-[1,3,4]oxdiazol-2-on-trifluoracetat | 509 | 508,2 |
| 142 | 3-[4-(4-Hexyloxy-benzylamino)-phenyl]-5-methoxi-3H-[1,3,4]oxdiazol-2-on-trifluoracetat | 512 | 511,5 |
| 143 | 5-Methoxi-3-[4-[3-(3-trifluormethyl-phenoxy)-benzylamino]-phenyl]-3H-[1,3,4]oxdiazol-2-on-trifluoracetat | 572 | 571,4 |
| | | | |
| 144 | 3-[4-[(2-Chlor-chinolin-3-ylmethyl)-amino]-phenyl]-5-methoxi-3H-[1,3,4]oxdiazol-2-on-trifluoracetat | 497 | 496,8 |
| 145 | 3-Methoxi-5-[[4-(5-methoxi-2-oxo-[1,3,4joxdiazol-3-yl)-phenylamino]-methyl]-pyridine-2-carbonsäure methylestertrifluoracetat | 501 | 500,4 |
| 146 | Benzolsulfonsäure-4-[[4-(5-methoxi-2-oxo-[1,3,4]oxdiazol-3-yl)-phenylamino]-methyl]-phenyl ester | 454 | 453,5 |
| 147 | 2-(2,6-Dimethyl-4-methylsulfanyl-phenoxy)-N-[3-(5-methoxi-2-oxo-[1,3,4]oxdiazol-3-yl)-phenyl]-acetamid | 416 | 415,5 |
| 148 | 1-(2,4-Difluor-phenyl)-3-[4-(5-methoxi-2-oxo-[1,3,4]oxdiazol-3-yl)-phenyl]-harnstoff | 363 | 362,3 |
| 149 | 1-[4-(5-Methoxi-2-oxo-[1,3,4]oxdiazol-3-yl)-phenyl]-3-(4-phenoxyphenyl)-harnstoff | 419 | 418,4 |
| 150 | 1-(2,6-Difluor-phenyl)-3-[4-(5-methoxi-2-oxo-[1,3,4]oxdiazol-3-yl)-phenyl]-harnstoff | 363 | 362,3 |
| 151 | 1-Butyl-3-[4-(5-methoxi-2-oxo-[1,3,4]oxdiazol-3-yl)-phenyl]-harnstoff | 307 | 306,3 |
| 152 | 1-(2-Ethoxi-phenyl)-3-[4-(5-methoxi-2-oxo-[1,3,4]oxdiazol-3-yl)-phenyl]-harnstoff | 371 | 370,4 |
| 153 | 1-(2,6-Dibromo-4-fluor-phenyl)-3-[4-(5-methoxi-2-oxo-[1,3,4]oxdiazol-3-yl)-phenyl]-harnstoff | 503 | 502,1 |
| 154 | 1-(4-Butoxy-phenyl)-3-[4-(5-methoxi-2-oxo-[1,3,4]oxdiazol-3-yl)-phenyl]-harnstoff | 399 | 398,4 |
| 155 | 1-[4-(5-Methoxi-2-oxo-[1,3,4]oxdiazol-3-yl)-phenyl]-3-(4-trifluormethoxi-phenyl)-harnstoff | 411 | 410,3 |
| 156 | 1-Benzyl-3-[4-(5-methoxi-2-oxo-[1,3,4]oxdiazol-3-yl)-phenyl]-harnstoff | 341 | 340,3 |
| 157 | 1-(3-Fluor-phenyl)-3-[4-(5-methoxi-2-oxo-[1,3,4]oxdiazol-3-yl)-phenyl]-harnstoff | 345 | 344,3 |
| 158 | 6-[3-[4-(5-Methoxi-2-oxo-[1,3,4]oxdiazol-3-yl)-phenyl]-ureido]-hexansäure ethyl ester | 393 | 392,4 |
| 159 | 1-Biphenyl-4-yl-3-[4-(5-methoxi-2-oxo-[1,3,4]oxdiazol-3-yl)-phenyl]-harnstoff | 403 | 402,4 |
| 160 | 2-[3-[4-(5-Methoxi-2-oxo-[1,3,4]oxdiazol-3-yl)-phenyl]-ureido]-benzoesäure butyl ester | 427 | 426,4 |
| 161 | 5-Methoxi-3-[3-(7-methoxi-3,7-dimethyl-octylamino)-phenyl]-3H-[1,3,4]oxdiazol-2-on-trifluoracetat | 492 | 491,5 |
| 162 | 5-Methoxi-3-[3-[(thiophen-2-ylmethyl)-amino]-phenyl]-3H-[1,3,4]oxdiazol-2-on-trifluoracetat | 418 | 417,4 |
| 163 | 3-(3-Hexylamino-phenyl)-5-methoxi-3H-[1,3,4]oxdiazol-2-on-trifluoracetat | 406 | 405,4 |
| 164 | 5-Methoxi-3-[3-(3-phenyl-propylamino)-phenyl]-3H-[1,3,4]oxdiazol-2-on-trifluoracetat | 440 | 439,4 |
| 165 | 5-Methoxi-3-(3-undecylamino-phenyl)-3H-[1,3,4]oxdiazol-2-on-trifluoracetat | 476 | 475,5 |
| 166 | 5-Methoxi-3-[3-[3-(3-trifluormethyl-phenoxy)-benzylamino]-phenyl]-3H-[1,3,4]oxdiazol-2-on-trifluoracetat | 572 | 571,4 |
| | | | |
| 167 | 3-[3-[(2-Chlor-chinolin-3-ylmethyl)-amino]-phenyl]-5-methoxi-3H-[1,3,4]oxdiazol-2-on-trifluoracetat | 497 | 496,8 |
| 168 | 4-Fluor-benzolsulfonic acid 4-[[3-(5-methoxi-2-oxo-[1,3,4]oxdiazol-3-yl)-phenylamino]-methyl]-phenylester-trifluoracetat | 586 | 585,5 |
| | | | |
| 169 | 5-Methoxi-3-[3-(3,4,5-trifluor-benzylamino)-phenyl]-3H-[1,3,4]oxdiazol-2-on-trifluoracetat | 466 | 465,3 |
| 170 | 3-[3-(3,5-Bis-trifluormethyl-benzylamino)-phenyl]-5-methoxi-3H-[1,3,4]oxdiazol-2-on-trifluoracetat | 548 | 547,3 |
| 171 | 3-(3-Dec-4-enylamino-phenyl)-5-methoxi-3H-[1,3,4]oxdiazol-2-on-trifluoracetat | 460 | 459,5 |
| 172 | 3-[3-(3-Cyclopentyl-2-phenethyloxy-benzylamino)-phenyl]-5-methoxi-3H-[1,3,4]oxdiazol-2-on-trifluoracetat | 600 | 599,6 |
| 173 | 4-[[3-(5-Methoxi-2-oxo-[1,3,4]oxdiazol-3-yl)-phenylamino]-methyl]-benzonitril-trifluoracetat | 437 | 436,3 |
| 174 | 5-Methoxi-3-[3-[(6-methyl-pyridin-2-ylmethyl)-amino]-phenyl]-3H-[1,3,4]oxdiazol-2-on-trifluoracetat | 427 | 426,3 |
| 175 | 3-[3-(2-Benzyloxy-ethylamino)-phenyl]-5-methoxi-3H-[1,3,4]oxdiazol-2-on-trifluoracetat | 456 | 455,4 |
| 176 | 3-[3-(2,6-Difluor-benzylamino)-phenyl]-5-methoxi-3H-[1,3,4]oxdiazol-2-on-trifluoracetat | 448 | 447,3 |
| | | | |

| | | Fp. °C | |
|---|---|---|---|
| 177 | Dodecansäure [4-(5-methoxi-2-oxo-[1,3,4]oxdiazol-3-yl)-phenyl]-amid | 93 | |
| 178 | Octadec-9-ensäure [4-(5-methoxi-2-oxo-[1,3,4]oxdiazol-3-yl)-phenyl]-amid | 67 | |
| 179 | [4-(5-Methoxi-2-oxo-[1,3,4]oxdiazol-3-yl)-2-methyl-phenyl]-carbamic acid 2-methoxi-ethylester | 117 | |
| 180 | 1-(4-Hydroxi-cyclohexyl)-3-[4-(5-methoxi-2-oxo-[1,3,4]oxdiazol-3-yl)-2-methyl-phenyl]-harnstoff | 220 | |
| 181 | 1,1-Dibutyl-3-[4-(5-methoxi-2-oxo-[1,3,4]oxdiazol-3-yl)-2-methyl-phenyl]-harnstoff | Öl | |
| 182 | 5-Methoxi-benzofuran-2-carbonsäure-[4-(5-methoxi-2-oxo-[1,3,4]oxdiazol-3-yl)-2- methyl-phenyl]-amid | 199 | |
| 183 | 4-Methyl-piperazine-1-carboxilic acid [4-(5-methoxi-2-oxo-[1,3,4]oxdiazol-3-yl)-2-methyl-phenyl]-amid | Öl | |
| 184 | [4-(5-Methoxi-2-oxo-[1,3,4]oxdiazol-3-yl)-2-methyl-phenyl]-carbaminsäure-1-methyl- piperidin-4-ylester | 235 | |
| 185 | [4-(5-Methoxi-2-oxo-[1,3,4]oxdiazol-3-yl)-2-methyl-phenyl]-carbaminsäure-cyclohexylester | 163 | |
| 186 | 4-Benzyl-piperidin-1-carbonsäure-[4-(5-methoxi-2-oxo-[1,3,4]oxdiazol-3-yl)-2-methyl- phenyl]-amid | 146 | |
| 187 | 1-(2-Diisopropylamino-ethyl)-3-[4-(5-methoxi-2-oxo-[1,3,4]oxdiazol-3-yl)-2-methyl- phenyl]-harnstoff | 136 | |
| 188 | 4-(2-{3-[4-(5-Methoxi-2-oxo-[1,3,4]oxdiazol-3-yl)-2-methyl-phenyl]-ureido}-ethyl)- benzolsulfonamid | 200 | |
| 189 | 1-(1-Benzyl-piperidin-4-yl)-3-[4-(5-methoxi-2-oxo-[1,3,4]oxdiazol-3-yl)-2-methyl-phenyl]- harnstoff | 198 | |
| 190 | 1-(4-Isopropyl-phenyl)-3-[4-(5-methoxi-2-oxo-[1,3,4]oxdiazol-3-yl)-2-methyl-phenyl]- harnstoff | 200 | |
| 191 | 2-{3-[4-(5-Methoxi-2-oxo-[1,3,4]oxdiazol-3-yl)-2-methyl-phenyl]-ureido}-3-methyl-buttersäure | 246 | |
| 192 | [4-(5-Methoxi-2-oxo-[1,3,4]oxdiazol-3-yl)-2-methyl-phenyl]-carbaminsäure 1,2,3,4- tetrahydro-naphth-1-ylester | 159 | |
| 193 | [4-(5-Methoxi-2-oxo-[1,3,4]oxdiazol-3-yl)-2-methyl-phenyl]-carbaminsäure 1-phenyl-ethylester | Öl | |
| 194 | [4-(5-Methoxi-2-oxo-[1,3,4]oxdiazol-3-yl)-2-methyl-phenyl]-carbaminsäure 4-isopropyl- benzylester | 88 | |
| 195 | [4-(5-Methoxi-2-oxo-[1,3,4]oxdiazol-3-yl)-2-methyl-phenyl]-carbaminsäure 4- trifluormethoxi-benzylester | 82 | |
| 196 | [4-(5-Methoxi-2-oxo-[1,3,4]oxdiazol-3-yl)-2-methyl-phenyl]-carbaminsäure 3,5-dichloro- benzylester | 169 | |
| 197 | [4-(5-Methoxi-2-oxo-[1,3,4]oxdiazol-3-yl)-2-methyl-phenyl]-carbaminsäure biphenyl-2-ylmethylester | 138 | |
| 198 | 5-Chlor-benzofuran-2-carbonsäure-[4-(5-methoxi-2-oxo-[1,3,4]oxdiazol-3-yl)-2-methyl- phenyl]-amid | 210 | |
| 199 | 5-Chlor-benzofuran-2-carbonsäure-[4-(5-methoxi-2-oxo-[1,3,4]oxdiazol-3-yl)-phenyl]- amid | 209 | |

## Patentansprüche

1. Verbindungen der allgemeinen Formel 1 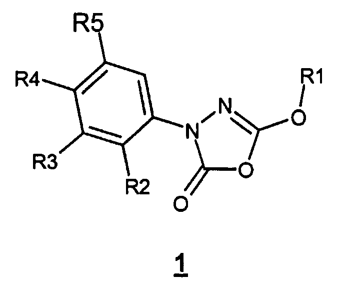 worin bedeuten:
R¹ C₁-C₆-Alkyl, C₃-C₉-Cycloalkyl, wobei beide Gruppen durch Phenyl, C₁-C₄-Alkyloxy, S-C₁-C₄-Alkyl, N(C₁-C₄-Alkyl)₂ ein- oder mehrfach substituiert sein können und Phenyl wiederum durch Halogen, C₁-C₄-Alkyl, C₁-C₄-Alkyloxy, Nitro, CF₃, ein- oder mehrfach substituiert sein kann; und
R², R³, R⁴ und R⁵ unabhängig voneinander Wasserstoff, Halogen, Nitro, C₁-C₄-Alkyl, C₁-C₉-Alkyloxy;
C₆-C₁₀-Aryl-C₁-C₄-alkyloxy, C₆-C₁₀-Aryloxy, C₆-C₁₀-Aryl, C₃-C₈-Cycloalkyl oder O-C₃-C₈-Cycloalkyl, die durch Halogen, CF₃, C₁-C₄-Alkyloxy oder C₁-C₄-Alkyl einfach, doppelt oder dreifach substituiert sein können;
2- Oxo-pyrrolidin-1-yl, 2,5-Dimethylpyrrol-1-yl oder NR⁶-A-R⁷,
mit der Maßgabe, dass R², R³, R⁴ und R⁵ nicht gleichzeitig Wasserstoff bedeuten und mindestens einer der Reste R², R³, R⁴ oder R⁵ für den Rest 2-Oxo-pyrrolidin-1-yl, 2,5-Dimethylpyrrol-1-yl oder NR⁶-A-R⁷steht, mit
R⁶ Wasserstoff, C₁-C₄-Alkyl oder C₆-C₁₀-Aryl-C₁-C₄-alkyl, wobei Aryl durch Halogen, CF₃, C₁-C₈-Alkyloxy oder C₁-C₄-Alkyl substituiert sein kann;
A eine einfache Bindung, COₙ, SOₙ oder CONH;
n 1 oder 2;
R⁷ Wasserstoff;
C₁-C₁₈-Alkyl oder C₂-C₁₈-Alkenyl, die ein- bis dreifach durch C₁-C₄-Alkyl, Halogen, CF₃, C₁-C₄-Alkyloxy, N(C₁-C₄-Alkyl)₂, -COOH, C₁-C₄-Alkyloxycarbonyl, C₆-C₁₂-Aryl, C₆-C₁₂-Aryloxy, C₆-C₁₂-Arylcarbonyl, C₆-C₁₀-Aryl-C₁-C₄-alkyloxy oder Oxo substituiert sein können, wobei Aryl wiederum durch Halogen, C₁-C₄-Alkyl, Aminosulfonyl oder Methylmercapto substituiert sein kann;
C₆-C₁₀-Aryl-C₁-C₄-alkyl, C₅-C₈-Cycloalkyl-C₁-C₄-alkyl, C₅-C₈-Cycloalkyl, C₆-C₁₀-Aryl-C₂-C₆-alkenyl, C₆-C₁₀-Aryl, Diphenyl, Diphenyl-C₁-C₄-alkyl, Indanyl, die durch C₁-C₁₈-Alkyl, C₁-C₁₈-Alkyloxy, C₃-C₈-Cycloalkyl, COOH, Hydroxy, C₁-C₄-Alkylcarbonyl, C₆-C₁₀-Aryl-C₁-C₄-alkyl, C₆-C₁₀-Aryl-C₁-C₄-alkyloxy, C₆-C₁₀-Aryloxy, Nitro, Cyano, C₆-C₁₀-Aryl, Fluorsulfonyl, C₁-C₆-Alkyloxycarbonyl, C₆-C₁₀-Arylsulfonyloxy, Pyridyl, NHSO₂-C₆-C₁₀-Aryl, Halogen, CF₃ oder OCF₃ ein- oder zweifach substituiert sein können, wobei Alkyl nochmals durch C₁-C₄-Alkyloxycarbonyl, CF₃ oder Carboxy substituiert sein kann und Aryl durch Halogen, CF₃ oder C₁-C₄-Alkyloxy;
oder die Gruppe Het-(CH₂)ᵣ-,
mit r = 0, 1, 2 oder 3 und Het = gesättigter oder ungesättigter 5-7-gliedriger Heterocyclus, der benzoanelliert und mit C₁-C₄-Alkyl, C₆-C₁₀-Aryl, Halogen, C₁-C₄-Alkyloxy, C₁-C₄-Alkyloxycarbonyl, C₆-C₁₀-Aryl-C₁-C₄-alkyl, C₆-C₁₀-Aryl-C₁-C₄-alkylmercapto oder Nitro substituiert sein kann, wobei benzoanelliertes Aryl wiederum durch Halogen, C₁-C₄-Alkyloxy oder CF₃ und Alkyl in Arylalkyl durch Methoxy und CF₃ substituiert sein kann,
sowie deren pharmakologisch verträglichen Salzen und Säureadditionssalzen.

2. Verbindungen der Formel 1 gemäß Anspruch 1, worin R¹ C₁-C₄-Alkyl bedeutet.

3. Verbindungen der Formel 1 gemäß Anspruch 1 oder 2, worin R¹ Methyl bedeutet.

4. Verbindungen der Formel 1 gemäß den Ansprüchen 1 bis 3, worin R⁵ Wasserstoff bedeutet.

5. Verbindungen der Formel 1 gemäß den Ansprüchen 1 bis 4, worin R² Wasserstoff, Halogen, C₁-C₄-Alkyl, C₁-C₉-Alkyloxy oder Amino bedeutet.

6. Verbindungen der Formel 1 gemäß den Ansprüchen 1 bis 5, worin R³ bedeutet Wasserstoff, C₁-C₄-Alkyl, C₆-C₁₀-Aryl-C₁-C₄-alkyloxy, das gegebenenfalls im Arylteil durch Halogen substituiert sein kann oder NR⁶-A-R⁷ mit
R⁶ = Wasserstoff oder Benzyl,
A = einfacher Bindung und
R⁷ = C₆-C₁₀-Aryl-C₁-C₄-alkyl, das durch Halogen, CF₃, Cyano, Phenyl-C₁-C₄alkyloxy, CF₃-Phenoxy, C₅-C₈-Cycloalkyl oder Fluorsulfonyloxy substituiert sein kann;
C₁-C₁₂-Alkyl, das durch C₁-C₄-Alkyloxy, Phenyl, CF₃ oder Phenyl-C₁-C₄alkyloxy substituiert sein kann;
C₂-C₁₂-Alkenyl oder der Gruppe Het-(CH₂)ᵣ-, mit r = 0 oder 1, und Het = gesättigter oder ungesättigter 5-7-gliedriger Heterocyclus, der benzoanelliert und mit C₁-C₄-Alkyl oder Halogen substituiert sein kann.

7. Verbindungen der Formel 1 gemäß den Ansprüchen 1 bis 6, worin R⁴ bedeutet Wasserstoff, 2-Oxo-pyrrolidin-1-yl, 2,5-Dimethylpyrrol-1-yl oder C₆-C₁₀-Aryl-C₁-C₄-alkyloxy, das durch Halogen substituiert sein kann.

8. Verbindungen der Formel 1 gemäß den Ansprüchen 1 bis 7, worin R⁴ NR⁶-A-R⁷ bedeutet, mit
R⁶ = Wasserstoff oder Methyl,
A = einfache Bindung und
R⁷ = Wasserstoff;
C₁-C₁₂-Alkyl, das ein- oder zweifach durch Halogen substituiert sein kann; C₂-C₁₈-Alkenyl, das ein- oder zweifach durch C₁-C₄-Alkyl oder C₁-C₄-Alkyloxycarbonyl substituiert sein kann;
C₆-C₁₀-Aryl-C₁-C₄-alkyl, das durch Halogen, C₁-C₆-Alkyloxy, CF₃, Cyano, C₅-C₆-Cycloalkyl, C₁-C₄-Alkyloxycarbonyl, C₆-C₁₀-Aryl-C₁-C₄-alkyl, C₆-C₁₀-Aryl-C₁-C₄-alkyloxy substituiert sein kann, wobei Aryl durch Halogen oder CF₃ nochmals substituiert sein kann;
C₅-C₈-Cycloalkyl-C₁-C₄-alkyl;
oder der Gruppe Het-(CH₂)ᵣ-,
mit r = 1, 2 oder 3 und Het = gesättigter oder ungesättigter 5-7-gliedriger Heterocyclus, der mit Halogen, C₁-C₄-Alkyloxy oder C₁-C₄-Alkyloxycarbonyl substituiert sein kann.

9. Verbindungen der Formel 1 gemäß den Ansprüchen 1 bis 8, worin R⁴ NR⁶-A-R⁷ bedeutet, mit
R⁶ = Wasserstoff,
A = -CO- und
R⁷ = C₁-C₁₈-Alkyl, das durch Halogen, Phenyl, Phenoxy, Phenylcarbonyl oder C₁-C₄-Alkyloxycarbonyl substituiert sein kann, wobei Phenoxy wiederum durch Methyl, Halogen oder Methylmercapto substituiert sein kann;
C₂-C₁₈-Alkenyl, das durch C₆-C₁₀-Aryl substituiert sein kann;
C₆-C₁₀-Aryl, das durch Halogen, C₁-C₈-Alkyl, Phenyl-C₁-C₄-alkyl, CF₃, OCF₃, Fluorsulfonyl, C₁-C₄-Alkyloxycarbonyl, Phenoxy substituiert sein kann, wobei Aryl wiederum durch C₁-C₄-Alkyloxy substituiert sein kann;
C₆-C₁₀-Aryl-C₁-C₄-alkyl, wobei Alkyl durch Methoxy oder CF₃ substituiert sein kann und Aryl durch Halogen;
oder der Gruppe Het-(CH₂)ᵣ-,
mit r = 0 und Het = gesättigter oder ungesättigter 5-7-gliedriger Heterocyclus, der benzoanelliert und mit C₁-C₄-Alkyl, Halogen, C₁-C₄-Alkyloxy, Halogenphenyl oder Halogen-benzylmercapto substituiert sein kann, wobei benzoanelliertes Aryl wiederum durch Halogen oder Methoxy substituiert sein kann.

10. Verbindungen der Formel 1 gemäß den Ansprüchen 1 bis 9, worin R⁴ NR⁶-A-R⁷ bedeutet, mit
R⁶ = Wasserstoff,
A = -CO₂- und
R⁷ = C₁-C₁₈-Alkyl, das durch CF₃ oder Phenyl substituiert ist; C₆-C₁₀-Aryl;
C₆-C₁₀-Aryl-C₁-C₄-alkyl, das durch C₁-C₄-Alkyl, Halogen, CF₃, oder OCF₃, Benzyloxy oder Phenyl substituiert ist;
oder der Gruppe Het-(CH₂)ᵣ-,
mit r = 0 oder 1 und Het = gesättigter oder ungesättigter 5-7-gliedriger Heterocyclus, der benzoanelliert und mit C₁-C₄-Alkyl oder Benzyl substituiert sein kann.

11. Verbindungen der Formel 1 gemäß den Ansprüchen 1 bis 10, worin R⁴ NR⁶-A-R⁷ bedeutet, mit
R⁶ = Wasserstoff,
A = -SO₂- und
R⁷ = C₁-C₆-Alkyl, das mit CF₃ substituiert sein kann;
C₂-C₄-Alkenyl, das durch Phenyl substituiert sein kann;
C₆-C₁₀-Aryl, das durch C₁-C₆-Alkyl, Halogen, C₁-C₄-Alkyloxy oder Benzyl substituiert sein kann ;
Diphenyl-C₁-C₄-alkyl, substituiert mit Halogen;
oder der Gruppe Het-(CH₂)ᵣ-,
mit r = 0 und Het = gesättigter oder ungesättigter 5-7-gliedriger Heterocyclus.

12. Verbindungen der Formel 1 gemäß den Ansprüchen 1 bis 11, worin R⁴ NR⁶-A-R⁷ bedeutet, mit
R⁶ = Wasserstoff,
A = -CO-NH- und
R⁷ = C₁-C₁₀-Alkyl, das durch C₁-C₄-Alkyloxycarbonyl, N(C₁-C₄-Alkyl)₂ oder Phenyl substituiert sein kann , das wiederum durch Halogen oder Aminosulfonyl substituiert sein kann;
C₆-C₁₀-Aryl, das durch C₁-C₆-Alkyl, C₁-C₆-Alkyloxy, C₁-C₆-Alkyloxycarbonyl, Phenoxy, OCF₃, Benzyl oder Pyridyl, wobei Alkyl nochmals durch C₁-C₄-Alkyloxycarbonyl oder Carboxy substituiert sein kann;
C₅-C₈-Cycloalkyl, das durch Hydroxy substituiert sein kann oder Indanyl;
oder der Gruppe Het-(CH₂)ᵣ-,
mit r = 0 oder 1 und Het = gesättigter oder ungesättigter 5-7-gliedriger Heterocyclus, der mit Benzyl substituiert sein kann.

13. Verfahren zur Herstellung der Verbindungen der Formel 1 gemäß den Ansprüchen 1 bis 12, **gekennzeichnet durch** 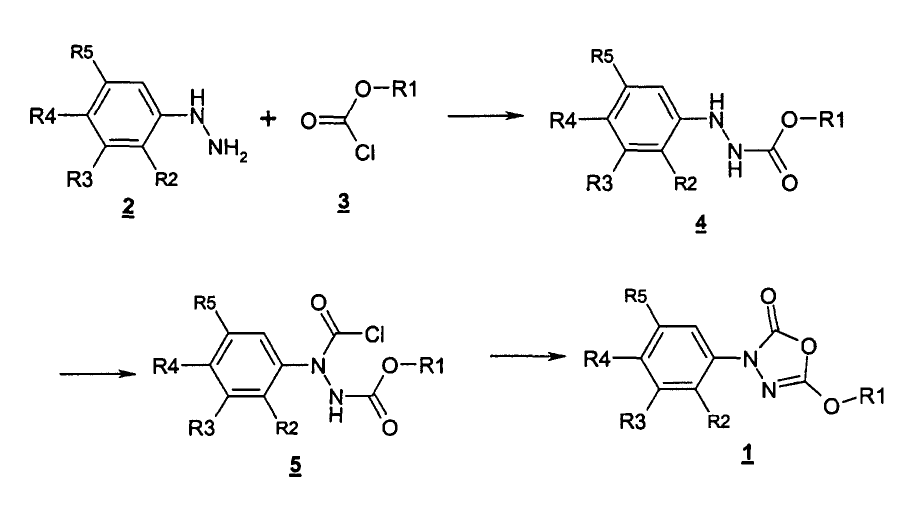 Umsetzung von Hydrazinen der allgemeinen Formel 2 mit Chlorameisensäureestern der Formel 3 oder anderen reaktiven Kohlensäureesterderivaten, worin R¹, R², R³, R⁴ und R⁵ wie in den Ansprüchen 1 bis 12 definiert sind, zu den Verbindungen der Formel 4, welche mit Phosgen, Carbonyldiimidazol, Diphosgen oder Triphosgen acyliert, zyklisiert und gegebenenfalls **durch** weitere chemische Modifikation der Reste R²-R⁵, wie beispielsweise **durch** Reduktion von Nitro- zu Aminoresten und anschließende Acylierung oder Alkylierung, zu den Verbindungen der Formel 1 umgesetzt werden.

14. Verbindungen der Formel 1 gemäß den Ansprüchen 1 bis 12 zur Anwendung in einem Arzneimittel mit hemmender Wirkung an der hormonsensitiven Lipase, HSL.

15. Verbindungen der Formel 1 gemäß den Ansprüchen 1 bis 12 zur Anwendung in einem Arzneimittel zur Behandlung des nicht insulinabhängigen Diabetes Mellitus oder des diabetischen Syndroms.

16. Arzneimittel zur Behandlung des nicht insulinabhängigen Diabetes Mellitus oder des diabetischen Syndroms enthaltend mindestens eine Verbindung der Formel 1 gemäß den Ansprüchen 1 bis 12.

## Claims

1. A compound of the formula 1 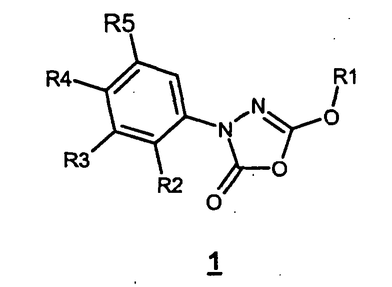 in which the meanings are:
R¹ C₁-C₆-alkyl, C₃-C₉-cycloalkyl, it being possible for both groups to be substituted one or more times by phenyl, C₁-C₄-alkyloxy, S-C₁-C₄-alkyl, N(C₁-C₄-alkyl)₂, and for phenyl in turn to be substituted one or more times by halogen, C₁-C₄-alkyl, C₁-C₄-alkyloxy, nitro, CF₃; and
R², R³, R⁴ and R⁵ independently of one another hydrogen, halogen, nitro, C₁-C₄-alkyl, C₁-C₉-alkyloxy;
C₆-C₁₀-aryl-C₁-C₄-alkyloxy, C₆-C₁₀-aryloxy, C₆-C₁₀-aryl, C₃-C₈-cycloalkyl or O-C₃-C₈-cycloalkyl, each of which may be substituted once, twice or three times by halogen, CF₃, C₁-C₄-alkyloxy or C₁-C₄-alkyl;
2-oxopyrrolidin-1-yl, 2,5-dimethylpyrrol-1-yl or NR⁶-A-R⁷,
with the proviso that R², R³, R⁴ and R⁵ are not simultaneously hydrogen, and at least one of the radicals R², R³, R⁴ or R⁵ is the radical 2-oxopyrrolidin-1-yl, 2,5-dimethylpyrrol-1-yl or NR⁶-A-R⁷, with
R⁶ hydrogen, C₁-C₄-alkyl or C₆-C₁₀-aryl-C₁-C₄-alkyl, where aryl may be substituted by halogen, CF₃, C₁-C₈-alkyloxy or C₁-C₄-alkyl;
A a single bond, COₙ, SOₙ or CONH;
n 1 or 2;
R⁷ hydrogen;
C₁-C₁₈-alkyl or C₂-C₁₈-alkenyl, each of which may be substituted once to three times by C₁-C₄-alkyl, halogen, CF_{3,} C₁-C₄-alkyloxy, N(C₁-C₄-alkyl)₂, -COOH, C₁-C₄-alkyloxycarbonyl, C₆-C₁₂-aryl, C₆-C₁₂-aryloxy, C₆-C₁₂-arylcarbonyl, C₆-C₁₀-aryl-C₁-C₄-alkyloxy or oxo,
where aryl in turn may be substituted by halogen, C₁-C₄-alkyl, aminosulfonyl or methylmercapto;
C₆-C₁₀-aryl-C₁-C₄-alkyl, C₅-C₈-cycloalkyl-C₁-C₄-alkyl, C₅-C₈-cycloalkyl, C₆-C₁₀-aryl-C₂-C₆-alkenyl, C₆-C₁₀-aryl, diphenyl, diphenyl-C₁-C₄-alkyl, indanyl, each of which may be substituted once or twice by C₁-C₁₈-alkyl, C₁-C₁₈-alkyloxy, C₃-C₈-cycloalkyl, COOH, hydroxyl, C₁-C₄-alkylcarbonyl, C₆-C₁₀-aryl-C₁-C₄-alkyl, C₆-C₁₀-aryl-C₁-C₄-alkyloxy, C₆-C₁₀-aryloxy, nitro, cyano, C₆-C₁₀-aryl, fluorosulfonyl, C₁-C₆-alkyloxycarbonyl, C₆-C₁₀-arylsulfonyloxy, pyridyl, NHSO₂-C₆-C₁₀-aryl, halogen, CF₃ or OCF₃, where alkyl may be substituted again by C₁-C₄-alkyloxycarbonyl, CF₃ or carboxyl, and aryl by halogen, CF₃ or C₁-C₄-alkyloxy;
or the group Het-(CH₂)ᵣ-,
with r = 0, 1, 2 or 3 and Het = saturated or unsaturated 5-7-membered heterocycle which may be benzo-fused and substituted by C₁-C₄-alkyl, C₆-C₁₀-aryl, halogen, C₁-C₄-alkyloxy, C₁-C₄-alkyloxycarbonyl, C₆-C₁₀-aryl-C₁-C₄-alkyl, C₆-C₁₀-aryl-C₁-C₄-alkylmercapto or nitro, where benzo-fused aryl may in turn be substituted by halogen, C₁-C₄-alkyloxy or CF₃ and alkyl in arylalkyl by methoxy and CF₃,
and the pharmacologically suitable salts and acid addition salts thereof.

2. A compound of the formula 1 as claimed in claim 1, in which R¹ is C₁-C₄-alkyl.

3. A compound of the formula 1 as claimed in claim 1 or 2, in which R¹ is methyl.

4. A compound of the formula 1 as claimed in claims 1 to 3, in which R⁵ is hydrogen.

5. A compound of the formula 1 as claimed in claims 1 to 4, in which R² is hydrogen, halogen, C₁-C₄-alkyl, C₁-C₉-alkyloxy or amino.

6. A compound of the formula 1 as claimed in claims 1 to 5, in which R³ is hydrogen, C₁-C₄-alkyl, C₆-C₁₀-aryl-C₁-C₄-alkyloxy which may optionally be substituted in the aryl moiety by halogen, or is NR⁶-A-R⁷ with
R⁶ = hydrogen or benzyl,
A = single bond and
R⁷ = C₆-C₁₀-aryl-C₁-C₄-alkyl which may be substituted by halogen, CF₃, cyano, phenyl-C₁-C₄-alkyloxy, CF₃-phenoxy, C₅-C₈-cycloalkyl or fluorosulfonyloxy;
C₁-C₁₂-alkyl which may be substituted by C₁-C₄-alkyloxy, phenyl,
CF₃ or phenyl-C₁-C₄-alkyloxy;
C₂-C₁₂-alkenyl or the group Het-(CH₂)ᵣ-, with r = 0 or 1, and Het = saturated or unsaturated 5-7-membered heterocycle which may be benzo-fused and substituted by C₁-C₄-alkyl or halogen.

7. A compound of the formula 1 as claimed in claims 1 to 6, in which R⁴ is hydrogen, 2-oxopyrrolidin-1-yl, 2,5-dimethylpyrrol-1-yl or C₆-C₁₀-aryl-C₁-C₄-alkyloxy which may be substituted by halogen.

8. A compound of the formula 1 as claimed in claims 1 to 7, in which
R⁴ is NR⁶-A-R⁷, with
R⁶ = hydrogen or methyl,
A = single bond and
R⁷ = hydrogen;
C₁-C₁₂-alkyl which may be substituted once or twice by halogen; C₂-C₁₈-alkenyl which may be substituted once or twice by C₁-C₄-alkyl
or C₁-C₄-alkyloxycarbonyl;
C₆-C₁₀-aryl-C₁-C₄-alkyl which may be substituted by halogen, C₁-C₆-alkyloxy, CF₃, cyano, C₅-C₆-cycloalkyl, C₁-C₄-alkyloxycarbonyl, C₆-C₁₀-aryl-C₁-C₄-alkyl, C₆-C₁₀-aryl-C₁-C₄-alkyloxy, where aryl may again be substituted by halogen or CF₃;
C₅-C₈-cycloalkyl-C₁-C₄-alkyl;
or the group Het-(CH₂)ᵣ-,
with r = 1, 2 or 3 and Het = saturated or unsaturated 5-7-membered heterocycle which may be substituted by halogen, C₁-C₄-alkyloxy or C₁-C₄-alkyloxycarbonyl.

9. A compound of the formula 1 as claimed in claims 1 to 8, in which
R⁴ is NR⁶-A-R⁷, with
R⁶ = hydrogen,
A = -CO- and
R⁷ = C₁-C₁₈-alkyl which may be substituted by halogen, phenyl, phenoxy, phenylcarbonyl or C₁-C₄-alkyloxycarbonyl, where phenoxy in turn may be substituted by methyl, halogen or methylmercapto; C₂-C₁₈-alkenyl which may be substituted by C₆-C₁₀-aryl;
C₆-C₁₀-aryl which may be substituted by halogen, C₁-C₈-alkyl, phenyl-C₁-C₄-alkyl, CF₃, OCF₃, fluorosulfonyl, C₁-C₄-alkyloxycarbonyl, phenoxy, where aryl in turn may be substituted by C₁-C₄-alkyloxy;
C₆-C₁₀-aryl-C₁-C₄-alkyl where alkyl may be substituted by methoxy or CF₃ and aryl by halogen;
or the group Het-(CH₂)ᵣ-,
with r = 0 and Het = saturated or unsaturated 5-7-membered heterocycle which may be benzo-fused and substituted by C₁-C₄-alkyl, halogen, C₁-C₄-alkyloxy, halophenyl or halobenzylmercapto,
where benzo-fused aryl may in turn be substituted by halogen or methoxy.

10. A compound of the formula 1 as claimed in claims 1 to 9, in which
R⁴ is NR⁶-A-R⁷, with
R⁶ = hydrogen,
A = -CO₂- and
R⁷ = C₁-C₁₈-alkyl which is substituted by CF₃ or phenyl;
C₆-C₁₀-aryl;
C₆-C₁₀-aryl-C₁-C₄-alkyl which is substituted by C₁-C₄-alkyl, halogen, CF₃ or OCF₃, benzyloxy or phenyl;
or the group Het-(CH₂)ᵣ-,
with r = 0 or 1 and Het = saturated or unsaturated 5-7-membered heterocycle which may be benzo-fused and substituted by C₁-C₄-alkyl or benzyl.

11. A compound of the formula 1 as claimed in claims 1 to 10, in which
R⁴ is NR⁶-A-R⁷, with
R⁶ = hydrogen,
A = -SO₂- and
R⁷ = C₁-C₆-alkyl which may be substituted by CF₃; C₂-C₄-alkenyl which may be substituted by phenyl;
C₆-C₁₀-aryl which may be substituted by C₁-C₆-alkyl, halogen, C₁-C₄-alkyloxy or benzyl;
diphenyl-C₁-C₄-alkyl substituted by halogen;
or the group Het-(CH₂)ᵣ-,
with r = 0 and Het = saturated or unsaturated 5-7-membered heterocycle.

12. A compound of the formula 1 as claimed in claims 1 to 11, in which
R⁴ is NR⁶-A-R⁷, with
R⁶ = hydrogen,
A = -CO-NH- and
R⁷ = C₁-C₁₀-alkyl which may be substituted by C₁-C₄-alkyloxycarbonyl, N(C₁-C₄-alkyl)₂ or phenyl which may in turn be substituted by halogen or aminosulfonyl;
C₆-C₁₀-aryl which may be substituted by C₁-C₆-alkyl, C₁-C₆-alkyloxy, C₁-C₆-alkyloxycarbonyl, phenoxy, OCF₃, benzyl or pyridyl, where alkyl may again be substituted by C₁-C₄-alkyloxycarbonyl or carboxyl;
C₅-C₈-cycloalkyl which may be substituted by hydroxyl, or indanyl;
or the group Het-(CH₂)ᵣ-,
with r = 0 or 1 and Het = saturated or unsaturated 5-7-membered heterocycle which may be substituted by benzyl.

13. A process for preparing compounds of the formula 1 as claimed in claims 1 to 12, which comprises 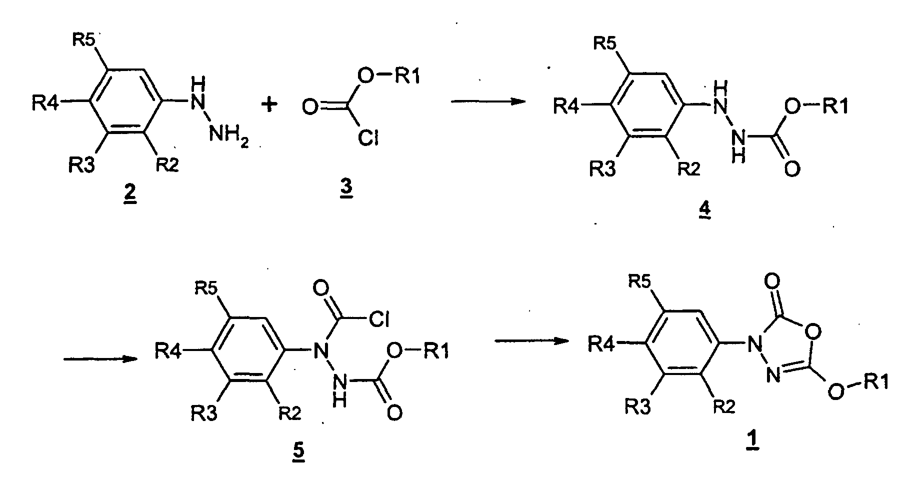 reacting hydrazines of the formula 2 with chloroformic esters of the formula 3 or other reactive carbonic ester derivatives in which R¹, R², R³, R⁴ and R⁵ are as defined in claims 1 to 12 to give the compounds of the formula 4, which are acylated with phosgene, carbonyldiimidazole, diphosgene or triphosgene, cyclized and, where appropriate, converted by further chemical modification of the radicals R²-R⁵ such as, for example; by reduction of nitro to amino radicals and subsequent acylation or alkylation into the compounds of the formula 1.

14. A compound of the formula 1 as claimed in claims 1 to 12 for use in a pharmaceutical with an inhibitory effect on hormone-sensitive lipase, HSL.

15. A compound of the formula 1 as claimed in claims 1 to 12 for use in a pharmaceutical for treating non-insulin-dependent diabetes mellitus or diabetic syndrome.

16. A pharmaceutical for treating non-insulin-dependent diabetes mellitus or diabetic syndrome comprising at least one compound of the formula 1 as claimed in claims 1 to 12.

## Revendications

1. Composés de formule générale 1 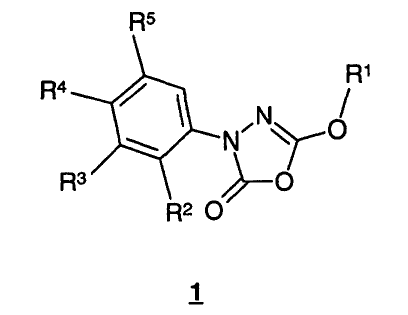 où :
R¹ représente alkyle en C₁-C₆, cycloalkyle en C₃-C₉, où les deux groupes peuvent être substitués une ou plusieurs fois par phényle, alkyloxy en C₁-C₄, S-alkyle en C₁-C₄, N(alkyle en C₁-C₄)₂, et phényle qui peut être substitué lui aussi une ou plusieurs fois par halogène, alkyle en C₁-C₄, alkyloxy en C₁-C₄, nitro, CF₃ ; et
R², R³, R⁴ et R⁵ représente indépendamment les uns des autres l'hydrogène, halogène, nitro, alkyle en C₁-C₄, alkyloxy en C₁-C₉ ; aryle en C₆-C₁₀-alkyloxy en C₁-C₄, aryloxy en C₆-C₁₀, aryle en C₆-C₁₀, cycloalkyle en C₃-C₈ ou O-cycloalkyle en C₃-C₈, qui peuvent être substitués une, deux ou trois fois par halogène, CF₃, alkyloxy en C₁-C₄ ou alkyle en C₁-C₄ ;
2-oxo-pyrrolidin-1-yle, 2,5-diméthylpyrrol-1-yle ou NR⁶-A-R⁷, à condition que R², R³, R⁴ et R⁵ ne représentent pas simultanément l'hydrogène et qu'au moins l'un des restes R², R³, R⁴ et R⁵ représente le reste 2-oxo-pyrrolidin-1-yle, 2,5-diméthylpyrrol-1-yle ou NR⁶-A-R⁷, où
R⁶ représente l'hydrogène, alkyle en C₁-C₄ ou aryle en C₆-C₁₀-alkyle en C₁-C₄, où aryle peut être substitué par halogène, CF₃, alkyloxy en C₁-C₈ ou alkyle en C₁-C₄ ;
A représente une simple liaison, COₙ, SOₙ ou CONH ;
n représente 1 ou 2 ;
R⁷ représente l'hydrogène ;
alkyle en C₁-C₁₈ ou alcényle en C₂-C₁₈, qui peuvent être substitués une à trois fois par alkyle en C₁-C₄, halogène, CF₃, alkyloxy en C₁-C₄, N(alkyle en C₁-C₄)₂, -COOH, alkyle en C₁-C₄-oxycarbonyle, aryle en C₆-C₁₂, aryloxy en C₆-C₁₂, aryle en C₆-C₁₂-carbonyle, aryle en C₆-C₁₀-alkyloxy en C₁-C₄ ou oxo, où aryle peut être substitué lui aussi par halogène, alkyle en C₁-C₄, aminosulfonyle ou méthylmercapto ;
aryle en C₆-C₁₀-alkyle en C₁-C₄, cycloalkyle en C₅-C₈-alkyle en C₁-C₄, cycloalkyle en C₅-C₈, aryle en C₆-C₁₀-alcényle en C₂-C₆, aryle en C₆-C₁₀, diphényle, diphényl-alkyle en C₁-C₄, indanyle, qui peuvent être substitués une ou deux fois par alkyle en C₁-C₁₈, alkyloxy en C₁-C₁₈, cycloalkyle en C₃-C₈, COOH, hydroxyle, alkyle en C₁-C₄-carbonyle, aryle en C₆-C₁₀-alkyle en C₁-C₄, aryle en C₆-C₁₀-alkyloxy en C₁-C₄, aryloxy en C₆-C₁₀, nitro, cyano, aryle en C₆-C₁₀, fluorosulfonyle, alkyle en C₁-C₆-oxycarbonyle, aryle en C₆-C₁₀-sulfonyloxy, pyridyle, NHSO₂-aryle en C₆-C₁₀, halogène, CF₃ ou OCF₃, où alkyle peut être substitué encore par alkyle en C₁-C₄-oxycarbonyle, CF₃ ou carboxyle et aryle peut être substitué par halogène, CF₃ ou alkyloxy en C₁-C₄ ;
ou le groupe Het-(CH₂)ᵣ-,
avec r = 0, 1, 2 ou 3 et Het = hétérocycle à 5-7 chaînons saturé ou insaturé, qui peut être benzocondensé et substitué par alkyle en C₁-C₄, aryle en C₆-C₁₀ halogène, alkyloxy en C₁-C₄, alkyle en C₁-C₄-oxycarbonyle, aryle en C₆-C₁₀-alkyle en C₁-C₄, aryle en C₆-C₁₀-alkyle en C₁-C₄-mercapto ou nitro, où aryle benzocondensé peut être substitué lui aussi par halogène, alkyloxy en C₁-C₄ ou CF₃ et alkyle dans arylalkyle peut être substitué par méthoxy et CF₃,
ainsi que leurs sels et sels d'addition d'acide pharmacologiquement acceptables.

2. Composés de formule 1 selon la revendication 1, où R¹ représente alkyle en C₁-C₄.

3. Composés de formule 1 selon la revendication 1 ou 2, où R¹ représente méthyle.

4. Composés de formule 1 selon les revendications 1 à 3, où R⁵ représente l'hydrogène.

5. Composés de formule 1 selon les revendications 1 à 4, où R² représente l'hydrogène, halogène, alkyle en C₁-C₄, alkyloxy en C₁-C₉ ou amino.

6. Composés de formule 1 selon les revendications 1 à 5, où R³ représente l'hydrogène, alkyle en C₁-C₄, aryle en C₆-C₁₀-alkyloxy en C₁-C₄, qui peut éventuellement être substitué par halogène dans la partie aryle, ou NR⁶-A-R⁷ avec
R⁶ = l'hydrogène ou benzyle,
A = simple liaison et
R⁷ = aryle en C₆-C₁₀-alkyle en C₁-C₄, qui peut être substitué par halogène, CF₃, cyano, phényl-alkyloxy en C₁-C₄, CF₃-phénoxy, cycloalkyle en C₅-C₈ ou fluorosulfonyloxy ;
alkyle en C₁-C₁₂, qui peut être substitué par alkyloxy en C₁-C₄, phényle, CF₃ ou phényl-alkyloxy en C₁-C₄ ;
alcényle en C₂-C₁₂ ou le groupe Het-(CH₂)ᵣ-, avec r = 0 ou 1, et Het = hétérocycle à 5-7 chaînons saturé ou insaturé, qui peut être benzocondensé et substitué par alkyle en C₁-C₄ ou halogène.

7. Composés de formule 1 selon les revendications 1 à 6, où R⁴ représente l'hydrogène, 2-oxo-pyrrolidin-1-yle, 2,5-diméthylpyrrol-1-yle ou aryle en C₆-C₁₀-alkyloxy en C₁-C₄, qui peut être substitué par halogène.

8. Composés de formule 1 selon les revendications 1 à 7, où R⁴ représente NR⁶-A-R⁷, avec
R⁶ = l'hydrogène ou méthyle,
A = simple liaison et
R⁷ = l'hydrogène ;
alkyle en C₁-C₁₂, qui peut être substitué une ou deux fois par halogène ;
alcényle en C₂-C₁₈, qui peut être substitué une ou deux fois par alkyle en C₁-C₄ ou alkyle en C₁-C₄-oxycarbonyle ;
aryle en C₆-C₁₀-alkyle en C₁-C₄, qui peut être substitué encore par halogène, alkyloxy en C₁-C₆, CF₃, cyano, cycloalkyle en C₅-C₆, alkyle en C₁-C₄-oxycarbonyle, aryle en C₆-C₁₀-alkyle en C₁-C₄, aryle en C₆-C₁₀-alkyloxy en C₁-C₄, où aryle peut être substitué par halogène ou CF₃ ;
cycloalkyle en C₅-C₈-alkyle en C₁-C₄ ;
ou le groupe Het-(CH₂)ᵣ-,
avec r = 1, 2 ou 3 et Het = hétérocycle à 5-7 chaînons saturé ou insaturé, qui peut être substitué par halogène, alkyloxy en C₁-C₄ ou alkyloxy en C₁-C₄-carbonyle.

9. Composés de formule 1 selon les revendications 1 à 8, où R⁴ représente NR⁶-A-R⁷, avec
R⁶ = l'hydrogène,
A = -CO- et
R⁷ = alkyle en C₁-C₁₈, qui peut être substitué par halogène, phényle, phénoxy, phénylcarbonyle ou alkyle en C₁-C₄-oxycarbonyle, où phénoxy peut être substitué aussi par méthyle, halogène ou méthylmercapto ;
alcényle en C₂-C₁₈, qui peut être substitué par aryle en C₆-C₁₀ ;
aryle en C₆-C₁₀, qui peut être substitué par halogène, alkyle en C₁-C₈, phényl-alkyle en C₁-C₄, CF₃, OCF₃, fluorosulfonyle, alkyle en C₁-C₄-oxycarbonyle, phénoxy, où aryle peut être substitué aussi par alkyloxy en C₁-C₄ ;
aryle en C₆-C₁₀-alkyle en C₁-C₄ où alkyle peut être substitué par méthoxy ou CF₃ et aryle peut être substitué par halogène ;
ou le groupe Het-(CH₂)ᵣ-,
avec r = 0 et Het = hétérocycle à 5-7 chaînons saturé ou insaturé, qui peut être benzocondensé et substitué par alkyle en C₁-C₄, halogène, alkyloxy en C₁-C₄, halogénophényle ou halogénobenzylmercapto, où aryle benzocondensé peut être substitué aussi par halogène ou méthoxy.

10. Composés de formule 1 selon les revendications 1 à 9, où R⁴ représente NR⁶-A-R⁷, avec
R⁶ = hydrogène,
A = -CO₂- et
R⁷ = alkyle en C₁-C₁₈, qui est substitué par CF₃ ou phényle ;
aryle en C₆-C₁₀ ;
aryle en C₆-C₁₀-alkyle en C₁-C₄, qui est substitué par alkyle en C₁-C₄, halogène, CF₃ ou OCF₃, benzyloxy ou phényle ;
ou le groupe Het-(CH₂)ᵣ-,
avec r = 0 ou 1 et Het = hétérocycle à 5-7 chaînons saturé ou insaturé, qui peut être benzocondensé et substitué par alkyle en C₁-C₄ ou benzyle.

11. Composés de formule 1 selon les revendications 1 à 10, où R⁴ représente NR⁶-A-R⁷, avec
R⁶ = hydrogène,
A = -SO₂- et
R⁷ = alkyle en C₁-C₆, qui peut être substitué par CF₃ ;
Alcényle en C₂-C₄, qui peut être substitué par phényle ;
aryle en C₆-C₁₀, qui peut être substitué par alkyle en C₁-C₆, halogène, alkyloxy en C₁-C₄ ou benzyle ;
diphényl-alkyle en C₁-C₄, substitué par halogène ;
ou le groupe Het-(CH₂)ᵣ-,
avec r = 0 et Het = hétérocycle à 5-7 chaînons saturé ou insaturé.

12. Composés de formule 1 selon les revendications 1 à 11, où R⁴ représente NR⁶-A-R⁷, avec
R⁶ = hydrogène,
A = -CO-NH- et
R⁷ = alkyle en C₁-C₁₀, qui peut être substitué par alkyle en C₁-C₄-oxycarbonyle, N(alkyle en C₁-C₄)₂ ou phényle, qui peut être substitué aussi par halogène ou aminosulfonyle ;
aryle en C₆-C₁₀, qui peut être substitué par alkyle en C₁-C₆, alkyloxy en C₁-C₆, alkyle en C₁-C₆-oxycarbonyle, phénoxy, OCF₃, benzyle ou pyridyle, où alkyle peut être substitué aussi par alkyle en C₁-C₄-oxycarbonyle ou carboxyle ;
cycloalkyle en C₅-C₈, qui peut être substitué par hydroxyle, ou indanyle ;
ou le groupe Het-(CH₂)ᵣ-,
avec r = 0 ou 1 et Het = hétérocycle à 5-7 chaînons saturé ou insaturé, qui peut être substitué par benzyle.

13. Procédé de préparation des composés de formule 1 selon les revendications 1 à 12, **caractérisé par** 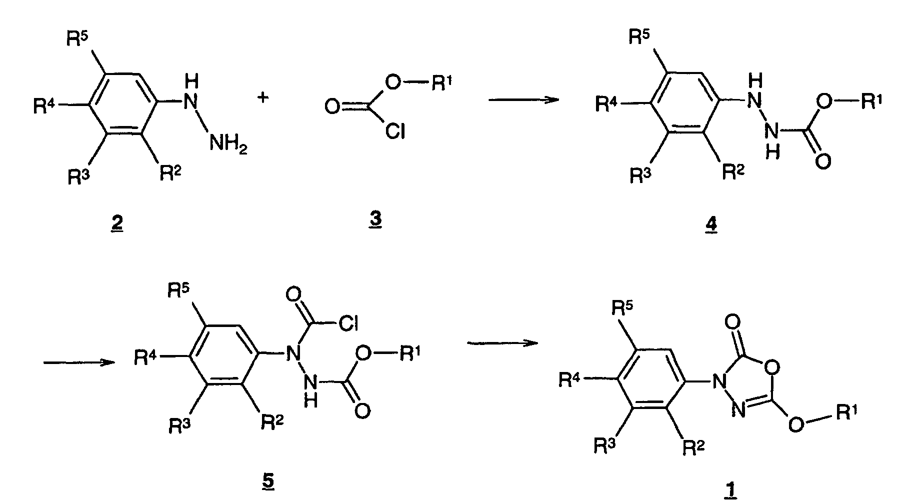 la réaction d'hydrazines de formule générale 2 avec des esters d'acide chloroformique de formule 3 ou d'autres dérivés d'esters d'acide carbonique réactifs, où R¹, R², R³, R⁴ et R⁵ sont définis comme dans les revendications 1 à 12, pour former les composés de formule 4 qui sont acylés avec le phosgène, le carbonyldiimidazole, le diphosgène ou le triphosgène, cyclisés et convertis en les composés de formule 1 éventuellement par une autre modification chimique des restes R²-R⁵, comme par exemple par réduction de restes nitro en restes amino puis acylation ou alkylation.

14. Composés de formule 1 selon les revendications 1 à 12 destinés à être utilisés dans un médicament ayant un effet inhibiteur sur la lipase hormonosensible HSL.

15. Composés de formule 1 selon les revendications 1 à 12, destinés à être utilisés dans un médicament pour le traitement du diabète sucré non insulinodépendant ou du syndrome diabétique.

16. Médicament pour le traitement du diabète sucré non insulinodépendant ou du syndrome diabétique contenant au moins un composé de formule 1 selon les revendications 1 à 12.
